# EUROPÄISCHE PATENTSCHRIFT

(11) **EP 0 726 250 B1**
(45) Veröffentlichungstag und Bekanntmachung des Hinweises auf die Patenterteilung: **02.11.2000**
(21) Anmeldenummer: 96101585.6
(22) Anmeldetag: 05.02.1996
(51) Int. Cl.: C07C 311/58, C07C 335/42, C07D 307/52, C07D 333/20, C07D 213/40, C07D 207/32, C07D 277/28, A61K 31/64, A61K 31/34, A61K 31/38, A61K 31/395

(54) **Substituierte Benzolsulfonylharnstoffe und -thioharnstoffe, Verfahren zu ihrer Herstellung und ihrer Verwendung zur Herstellung pharmazeutischer Präparate sowie sie enthaltende pharmazeutische Präparate**
Substituted benzenesulfonylureas and -thioureas, process for their preparation and their use in the production of pharmaceutical preparations as well as pharmaceutical preparations containing them
Benzènesulfonylurées et -thiourées substituées, procédé pour leur préparation et leur utilisation dans la production de préparations pharmaceutiques ainsi que préparations pharmaceutiques les contenant

(30) Priorität: 10.02.1995 DE 19504379
(43) Veröffentlichungstag der Anmeldung: 14.08.1996
(73) Patentinhaber: HOECHST AKTIENGESELLSCHAFT, 65929 Frankfurt am Main (DE)
(72) Erfinder: Englert, Heinrich Dr., D-65719 Hofheim (DE); Gerlach, Uwe, Dr, D-65795 Hattersheim (DE); Crause, Peter, Dr., D-63069 Offenbach (DE); Mania, Dieter, Dr., D-61462 Königstein (DE); Gögelein, Heinz, Dr., D-60259 Frankfurt (DE); Kaiser, Joachim, Dr., D-60431 Frankfurt (DE)

(56) Entgegenhaltungen:
- EP-A- 0 612 724
- BE-A- 754 454
- US-A- 3 494 936

## Beschreibung

Die Erfindung betrifft substituierte Benzolsulfonylharnstoffe und -thioharnstoffe der Formel I 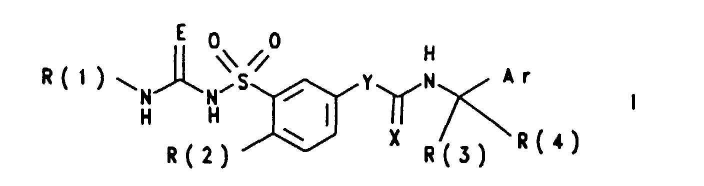 worin bedeuten:
- R(1): Wasserstoff, Alkyl mit 1, 2, 3, 4, 5 oder 6 C-Atomen oder Cycloalkyl mit 3, 4, 5 oder 6 C-Atomen;
- R(2): (C₁-C₈)-Ketten, in denen ein bis drei C-Atome durch Heteroatome O, NH, S ersetzt sein können, verzweigtes Alkyl mit 1, 2, 3, 4, 5 oder 6 C-Atomen, verzweigtes Alkoxy mit 1, 2, 3, 4, 5 oder 6 C-Atomen oder Cycloalkyl mit 3, 4, 5 oder 6 C-Atomen;
- R(3) und R(4): (gleich oder verschieden) Wasserstoff, Alkyl mit 1, 2, 3, 4, 5 oder 6 C-Atomen, oder gemeinsam eine Kette (CH₂)₂₋₅ bilden;
- E: Sauerstoff oder Schwefel;
- X: Sauerstoff oder Schwefel;
- Y: eine Kohlenwasserstoffkette der Formel [CR(5)₂]ₘ;
R(5) Wasserstoff oder Alkyl mit 1 oder 2 C-Atomen;
m 1 oder 2;
- Ar: Phenyl, Thienyl, Furyl, Pyrrolyl, Thiazolyl, Naphthyl, Pyridyl, das jeweils unsubstituiert oder substituiert ist mit ein bis 3 Substituenten ausgewählt aus der Gruppe bestehend aus Alkyl mit 1 oder 2 C-Atomen, Alkoxy mit 1 oder 2 C-Atomen, Cl, Br oder F;
und ihre physiologisch unbedenklichen Salze.

Der Begriff Alkyl beschreibt, sofern nicht anders angegeben, geradkettige oder verzweigte gesättigte Kohlenwasserstoffreste. Der Cycloalkylrest kann zusätzlich einen Alkylsubstituenten tragen. Als Halogensubstituent sind die Elemente Fluor, Chlor, Brom und lod einsetzbar.
Weiterhin können Verbindungen mit Chiralitätszentren, etwa in den Alkylketten Y, R(2), R(3) und R(4) auftreten. In diesem Fall gehören sowohl die einzelnen Antipoden für sich, als auch eine Mischung der beiden Enantiomere in unterschiedlichen Verhältnissen, sowie die dazugehörigen Mesoverbindungen oder Mischungen aus Mesoverbindungen, den Enantiomeren oder Diastereomeren zur Erfindung.

Ähnliche Sulfonylharnstoffe mit blutzuckersenkender Wirkung sind aus der belgischen Patentschrift 754 454 bekannt. Bestimmte Sulfonylharnstoffe und -thioharnstoffe mit deutlich verringerter blutzuckersenkender Wirkung, die sich strukturell von den Verbindungen der vorliegenden Erfindung unterscheiden, werden in der EP-A-612 724 beschrieben.

Die Verbindungen der Formel I dienen als Arzneimittelwirkstoffe in der Human- und Veterinärmedizin. Ferner können sie als Zwischenprodukte zur Herstellung weiterer Arzneimittelwirkstoffe verwendet werden.

Bevorzugt sind Verbindungen der Formel I, worin bedeuten:
- R(1): Wasserstoff, Alkyl mit 1, 2, 3, 4, 5 oder 6 C-Atomen oder Cycloalkyl mit 3, 4, 5 oder 6 C-Atomen;
- R(2): geradkettiges oder verzweigtes Alkyl mit 1, 2, 3, 4, 5 oder 6 C-Atomen, geradkettiges oder verzweigtes Alkoxy mit 1, 2, 3, 4, 5 oder 6 C-Atomen oder Cycloalkyl mit 3, 4, 5 oder 6 C-Atomen;
- R(3) und R(4): (gleich oder verschieden) Wasserstoff, Alkyl mit 1, 2, 3, 4, 5 oder 6 C-Atomen, oder gemeinsam eine Kette (CH₂)₂₋₅ bilden;
- E: Sauerstoff oder Schwefel;
- X: Sauerstoff oder Schwefel;
- Y: eine Kohlenwasserstoffkette der Formel [CR(5)₂]ₘ;
R(5) Wasserstoff oder Alkyl mit 1 oder 2 C-Atomen;
m 1 oder 2;
- Ar: Phenyl, Thienyl, Furyl, Pyrrolyl, Thiazolyl, Naphthyl, Pyridyl, das jeweils unsubstituiert oder substituiert ist mit ein bis 3 Substituenten ausgewählt aus der Gruppe bestehend aus Alkyl mit 1 oder 2 C-Atomen, Alkoxy mit 1 oder 2 C-Atomen, Cl, Br oder F;
und ihre physiologisch unbedenklichen Salze.

Bevorzugt sind auch Verbindungen der Formel 1, worin bedeuten:
- R(1): Wasserstoff, Alkyl mit 1, 2, 3, 4, 5 oder 6 C-Atomen oder Cycloalkyl mit 3, 4, 5 oder 6 C-Atomen;
- R(2): (C₁-C₈)-Ketten, in denen ein bis drei C-Atome durch Heteroatome 0, NH, S ersetzt sein können, verzweigtes Alkyl mit 1, 2, 3, 4, 5 oder 6 C-Atomen, verzweigtes Alkoxy mit 1, 2, 3, 4, 5 oder 6 C-Atomen oder Cycloalkyl mit 3, 4, 5 oder 6 C-Atomen;
- R(3) und R(4): (gleich oder verschieden) Wasserstoff, Alkyl mit 1, 2, 3, 4, 5 oder 6 C-Atomen oder gemeinsam eine Kette (CH₂)_{2 - 5} bilden;
- E: Schwefel;
- X: Sauerstoff;
- Y: eine Kohlenwasserstoffkette der Formel [CR(5)₂]₁₋₂,
R(5) Wasserstoff oder Alkyl mit 1 oder 2 C-Atomen;
- Ar: Phenyl, Thenyl, Furyl, Pyrrolyl, Thiazolyl, Naphthyl, Pyridyl, das jeweils unsubstituiert oder substituiert ist mit ein bis 3 Substituenten ausgewählt aus der Gruppe bestehend aus Alkyl mit 1 oder 2 C-Atomen, Alkoxy mit 1 oder 2 C-Atomen, Cl, Br oder F;
und ihre physiologisch unbedenklichen Salze.

Besonders bevorzugt sind Verbindungen der Formel l, worin bedeuten:
- R(1): Wasserstoff, Alkyl mit 1, 2, 3, 4, 5 oder 6 C-Atomen oder Cycloalkyl mit 3, 4, 5 oder 6 C-Atomen;
- R(2): geradkettiges oder verzweigtes Alkyl mit 1, 2, 3, 4, 5 oder 6 C-Atomen, geradkettiges oder verzweigtes Alkoxy mit 1, 2, 3, 4, 5 oder 6 C-Atomen oder Cycloalkyl mit 3, 4, 5 oder 6 C-Atomen;
- R(3) und R(4): (gleich oder verschieden) Wasserstoff, Alkyl mit 1, 2, 3, 4, 5 oder 6 C-Atomen oder gemeinsam eine Kette (CH₂)_{2 - 5} bilden;
- E: Schwefel;
- X: Sauerstoff;
- Y: eine Kohlenwasserstoffkette der Formel [CR(5)₂]₁₋₂,
R(5) Wasserstoff oder Alkyl mit 1 oder 2 C-Atomen;
- Ar: Phenyl, Thenyl, Furyl, Pyrrolyl, Thiazolyl, Naphthyl, Pyridyl, das jeweils unsubstituiert oder substituiert ist mit ein bis 3 Substituenten ausgewählt aus der Gruppe bestehend aus Alkyl mit 1 oder 2 C-Atomen, Alkoxy mit 1 oder 2 C-Atomen, Cl, Br oder F;
und ihre physiologisch unbedenklichen Salze.

Ganz besonders bevorzugt sind Verbindungen der Formel l, worin bedeuten:
- R(1): Wasserstoff oder Alkyl mit 1 oder 2 C-Atomen;
- R(2): geradkettiges oder verzweigtes Alkoxy mit 1, 2, 3, 4, 5 oder 6 C-Atomen;
- R(3) und R(4): (gleich oder verschieden) Wasserstoff, Alkyl mit 1, 2, 3, 4, 5 oder 6 C-Atomen;
- E: Schwefel;
- X: Sauerstoff;
- Y: eine Kohlenwasserstoffkette der Formel (CH₂)₁₋₂;
- Ar: Phenyl, Thenyl, Furyl, Pyrrolyl, Thiazolyl, Naphthyl, Pyridyl, das jeweils unsubstituiert oder substituiert ist mit ein bis 3 Substituenten ausgewählt aus der Gruppe bestehend aus Alkyl mit 1 oder 2 C-Atomen, Alkoxy mit 1 oder 2 C-Atomen, Cl, Br und F;
und ihre physiologisch unbedenklichen Salze.

Des weiteren bilden eine bevorzugte Gruppe die Verbindungen der Formel l, worin bedeuten:
- R(1): Wasserstoff oder Alkyl mit 1 oder 2 C-Atomen;
- R(2): geradkettiges oder verzweigtes Alkoxy mit 1, 2, 3, 4, 5 oder 6 C-Atomen;
- R(3) und R(4): (gleich oder verschieden) Wasserstoff oder Methyl;
- E: Schwefel;
- X: Sauerstoff;
- Y: eine Kohlenwasserstoffkette der Formel (CH₂)₁₋₂;
- Ar: Thienyl, Furyl, Pyrrolyl, Thiazolyl, Naphthyl, Pyridyl, das jeweils unsubstituiert oder substituiert ist mit ein bis 3 Substituenten ausgewählt aus der Gruppe bestehend aus Alkyl mit 1 oder 2 C-Atomen, Alkoxy mit 1 oder 2 C-Atomen, Cl, Br oder F;
und ihre physiologisch unbedenklichen Salze.

Ebenso bilden eine bevorzugte Gruppe die Verbindungen der Formel l, worin bedeuten:
- R(1): Wasserstoff oder Alkyl mit 1 oder 2 C-Atomen;
- R(2): geradkettiges oder verzweigtes Alkoxy mit 1, 2, 3, 4, 5 oder 6 C-Atomen;
- R(3) und R(4): (gleich oder verschieden) Wasserstoff oder Methyl;
- E: Schwefel;
- X: Sauerstoff;
- Y: eine Kohlenwasserstoffkette der Formel (CH₂)₁₋₂;
- Ar: Phenyl, das unsubstituiert oder substituiert ist mit ein bis 3 Substituenten ausgewählt aus der Gruppe bestehend aus Alkyl mit 1 oder 2 C-Atomen, Alkoxy mit 1 oder 2 C-Atomen, Cl, Br oder F;
und ihre physiologisch unbedenklichen Salze.

Die Verbindungen der vorliegenden Erfindung sind wertvolle Arzneimittel zur Behandlung von Herzrhythmusstörungen unterschiedlichster Genese und zur Verhinderung des arrhythmisch bedingten, plötzlichen Herztodes und können daher als Antiarrhythmika Verwendung finden. Beispiele arrhythmischer Störungen des Herzens sind supraventrikuläre Rhythmusstörungen wie etwa Vorhof-Tachycardien, Vorhof-Flattern oder paroxysmale supraventriculäre Rhythmusstörungen oder ventrikuläre Rhythmusstörungen wie ventrikuläre Extrasystolen, insbesondere aber lebensbedrohende ventrikuläre Tachycardien oder das besonders gefährliche Kammerflimmern. Sie eignen sich insbesondere für solche Fälle wo Arrhythmien Folge einer Verengung eines Koronargefäßes sind wie sie beispielsweise bei Angina Pectoris oder während eines akuten Herzinfarkts oder als chronische Folge eines Herzinfarkts auftreten. Sie sind daher insbesondere bei Postinfarktpatienten zur Verhinderung des plötzlichen Herztodes geeignet. Weitere Krankheitsbilder, wo derartige Rhythmusstörungen und/oder der plötzliche, arrhythmisch bedingte Herztod eine Rolle spielen, sind beispielsweise die Herzinsuffizienz oder die Herzhypertrophie als Folge eines chronisch erhöhten Blutdrucks.
Darüberhinaus können die Verbindungen eine verminderte Kontraktilität des Herzens positiv beeinflussen. Hierbei kann es sich um ein krankheitsbedingtes Nachlassen der Herzkontraktilität handeln, wie beispielsweise bei Herzinsuffizienz, aber auch um akute Fälle wie Herzversagen bei Schockeinwirkungen. Ebenso kann bei einer Herztransplantation das Herz nach erfolgter Operation seine Leistungsfähigkeit rascher und zuverlässiger wieder aufnehmen. Gleiches gilt für Operationen am Herz, die eine vorübergehende Still-Legung der Herzaktivität durch cardioplegische Lösungen erforderlich machen.

Die Erfindung betrifft weiterhin ein Verfahren zur Herstellung der Verbindungen I dadurch gekennzeichnet, daß man
(a) aromatische Sulfonamide der Formel II 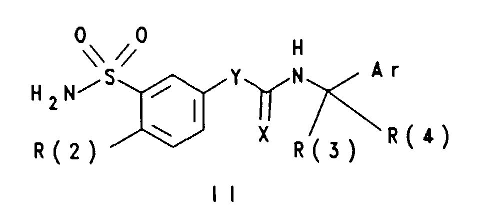 oder deren Salze der Formel III 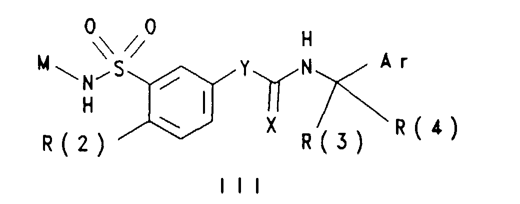 mit R(1)-substituierten Isocyanaten der Formel IV

   R(1)-N=C=O

   zu substituierten Benzolsulfonylharnstoffen I a (E = Sauerstoff) 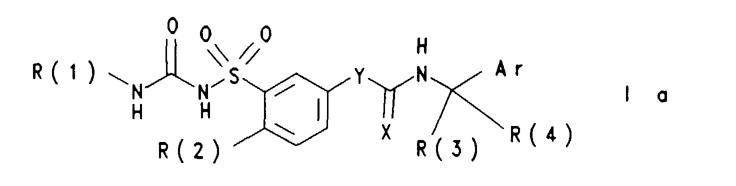 umsetzt.
   Als Kationen M in den Salzen der Formel III kommen Alkali-, Erdalkali-, Ammonium- sowie Tetraalkylammoniumionen in Betracht.
   Äquivalent zu den R(1)-substituierten Isocyanaten IV kann man R(1)-substituierte Carbamidsäureester, R(1)-substituierte Carbamidsäurehalogenide oder R(1)-substituierte Harnstoffe einsetzen.
(b) Unsubstituierte Benzolsulfonylharnstoffe I a [R(1) = H, E = O] kann man durch Umsetzung eines aromatischen Benzolsulfonamids der Formel II oder von dessen Salz III mit Trialkylsilylisocyanat oder Siliciumtetraisocyanat und Spaltung (z. B. Hydrolyse) der primären siliciumsubstituierten Benzolsulfonylharnstoffe herstellen.
   Weiterhin ist es möglich, ein Benzolsulfonamid II oder dessen Salz III durch Umsetzung mit Halogencyanen und Hydrolyse der primär entstehenden N-Cyanosulfonamide mit Mineralsäuren bei Temperaturen von 0°C bis 100°C in einen Benzolsulfonylharnstoff I a umzuwandeln.
(c) Ein Benzolsulfonylharnstoff I (mit E = Sauerstoff) läßt sich aus einem aromatischen einem Benzolsulfonamid II oder dessen Salz III mit einem R(1)-substituierten Trichloracetamid der Formel V 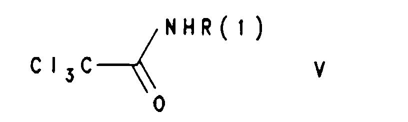 in Gegenwart einer Base in einem inerten Lösungsmittel nach Synthesis 1987, 734 - 735 bei Temperaturen von 25°C bis 150°C darstellen.
   Als Basen eignen sich zum Beispiel Alkalimetall- oder Erdalkalimetallhydroxide, -hydride, -amide oder auch -alkoholate, wie Natriumhydroxid, Kaliumhydroxid, Calciumhydroxyd, Natriumhydrid, Kaliumhydrid, Calciumhydrid, Natriumamid, Kaliumamid, Natriummethylat, Natriumethanolat, Kaliummethylat oder Kaliumethanolat. Als inerte Lösungsmittel eignen sich Ether wie Tetrahydrofuran, Dioxan, Ethylenglykoldimethylether (Diglyme), Ketone wie Aceton oder Butanon, Nitrile wie Acetonitril, Nitroverbindungen wie Nitromethan, Ester wie Ethylacetat, Amide wie Dimethylformamid (DMF) oder N-Methylpyrrolidon (NMP), Phosphorsäurehexamethyltriamid, Sulfoxide wie DMSO, Sulfone wie Sulfolan, Kohlenwasserstoffe wie Benzol, Toluol, Xylole. Weiterhin eignen sich auch Gemische dieser Lösungsmittel untereinander.
(d) Ein Benzolsulfonylthioharnstoff I (E = S) 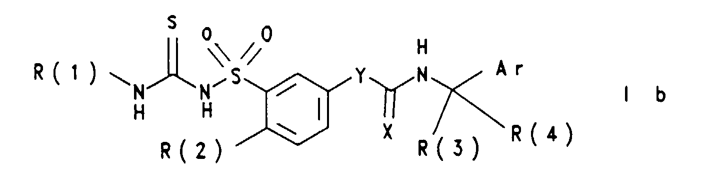 wird aus einem Benzolsulfonamid II oder dessen Salz III und einem R(1)-substituierten Isothiocyanat VI

   R(1)-N=C=S VI

   dargestellt.
   Einen unsubstituierten Benzolsulfonylthioharnstoff I b [R(1) = H, X = S] kann man durch Umsetzung eines aromatischen Benzolsulfonamids II oder von dessen Salz III mit Trimethylsilylisothiocyanat oder Siliciumtetraisothiocyanat und Spaltung (Hydrolyse) des primär entstehenden siliciumsubstituierten Benzolsulfonylharnstoffs herstellen. Weiterhin ist es möglich, ein aromatisches Benzolsulfonamid II oder dessen Salz III mit Benzoylisothiocyanat umzusetzen und den intermediären benzoylsubstituierten Benzolsulfonylthioharnstoff mit einer wäßrigen Mineralsäure zu I b [R(1) = H, E = S] umzusetzen. Ähnliche Verfahren sind beschrieben in J. Med. Chem. 1992, 35, 1137 - 1144. Eine weitere Variante besteht darin, die unter Verfahren 1 erwähnten N-Cyansulfonamide mit Schwefelwasserstoff umzusetzen.
(e) Einen substituierten Benzolsulfonylharnstoff der Formel I a (E = Sauerstoff) kann man durch eine Umwandlungsreaktion von einem Benzolsulfonylthioharnstoff der Struktur I b (E = S) herstellen. Der Ersatz des Schwefelatoms durch ein Sauerstoffatom im entsprechend substituierten Benzolsulfonylthioharnstoff kann beispielsweise mit Hilfe von Oxiden oder Salzen von Schwermetallen oder auch durch Anwendung von Oxidationsmitteln wie Wasserstoffperoxid, Natriumperoxid oder salpetriger Sdure, ausgeführt werden. Ein Thioharnstoff kann auch durch Behandlung mit Phosgen oder Phosphorpentachlorid entschwefelt werden. Als Zwischenverbindungen werden Chlorameisensäureamidine bzw. Carbodiimide erhalten, die zum Beispiel durch Verseifen oder Anlagerung von Wasser in die entsprechenden substituierten Benzolsulfonylharnstoffe überführt werden. Isothioharnstoffe verhalten sich bei der Entschwefelung wie Thioharnstoffe und können demzufolge ebenso als Ausgangsstoffe für diese Reaktionen dienen.
(f) Ein Benzolsulfonylharnstoff I a kann aus einem Benzolsulfonylhalogenid der Formel VII 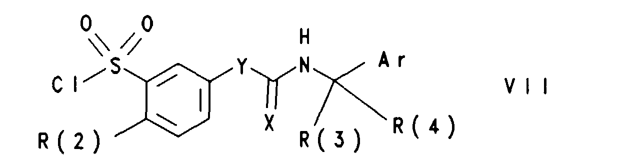 mit einem R(1)-substituierten Harnstoff oder einem R(1)-substituierten Bis(trialkylsilyl)harnstoff hergestellt werden. Weiterhin kann man das Sulfonsäurechlorid VII mit Parabansäure zu einer Benzolsulfonylparabansäure umsetzen, deren Hydrolyse mit Mineralsäuren den entsprechenden Benzolsulfonylharnstoff I a liefert.
(g) Ein Benzolsulfonylharnstoff I a läßt sich durch Umsetzung eines Amins der Formel R(1)-NH₂ mit einem Benzolsulfonylisocyanat der Formel VIII 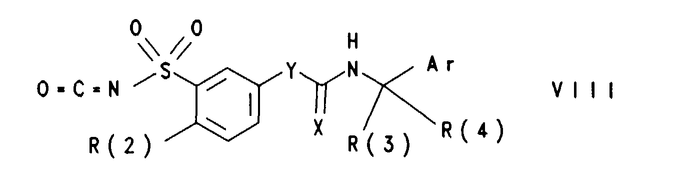 herstellen. Ebenso kann ein Amin R(1)-NH₂ mit einem Benzolsulfonylcarbamidsäureester, einem -carbamidsäurehalogenid oder einem Benzolsulfonylharnstoff I a [mit R(1) = H] zu den Verbindungen I a umgesetzt werden.
(h) Ein Benzolsulfonylthioharnstoff I b läßt sich durch Umsetzen von einem Amin der Formel R(1)-NH₂ mit einem Benzolsulfonylisothiocyanat der Formel IX 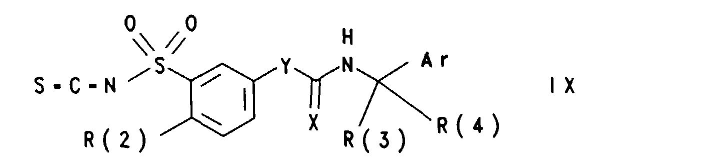 herstellen. Ebenso kann ein Amin R(1)-NH₂ mit einem Benzolsulfonylcarbamidsäurethioester, einem -carbamidsäurethiohalogenid zu den Verbindungen I b umgesetzt werden.
   Die Herstellung der Sulfonylisothiocyanate erfolgte durch Umsetzung eines entsprechenden Sulfonsäureamids mit äquimolaren Mengen Alkalihydroxid und Schwefelkohlenstoff in einem organischen Lösungsmittel, wie DMF, DMSO, N-Methylpyrrolidon. Das so erhaltene Di-alkalimetall-salz der Sulfonyldithiocarbaminsäure wird in einem inertem Lösungsmittel mit einem leichten Überschuß von Phosgen, bzw. Ersatzstoff desgleichen, wie Triphosgen, einem Chlorameisensäureester (2 Äquivalente) oder Thionylchlorid umgesetzt. Die so erhaltene Lösung des Sulfonylisothiocyanats kann direkt mit den entsprechenden Aminen oder Ammoniak umgesetzt werden.
(i) Ein entsprechend substituierter Benzolsulfenyl- oder -sulfinylharnstoff läßt sich mit einem Oxidationsmittel, wie Wasserstoffperoxid, Natriumperoxid oder salpetriger Säure, zum Benzolsulfonylharnstoff I a oxidieren.

Die Verbindungen I sowie deren physiologisch unbedenkliche Salze sind wertvolle Therapeutika, die sich nicht nur als Antiarrhythmika, sondern als Prophylaxe bei Störungen des cardiovaskulären Systems, Herzinsuffizienz, Herztransplantation oder cerebralen Gefäßerkrankungen an Menschen oder Säugetieren (zum Beispiel Affen, Hunde, Mäuse, Ratten, Kaninchen, Meerschweinchen und Katzen) eignen. Unter physiologisch unbedenklichen Salzen der Verbindungen I versteht man nach Remmington's Pharmaceutical Science, 17. Auflage, 1985, Seiten 14 - 18 Verbindungen der Formel XII, 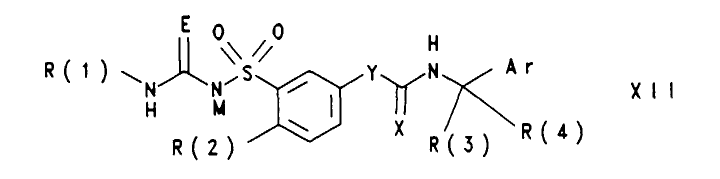 die sich aus nicht toxischen organischen und anorganischen Basen und den substituierten Benzolsulfonylharnstoffen I darstellen lassen. Bevorzugt werden hierbei Salze, in denen M in der Formel XII Natrium-, Kalium-, Rubidium-, Calcium-, Magnesium-, Ammoniumionen sind, sowie die Säureadditionsprodukte basischer Aminosäuren, wie zum Beispiel Lysin oder Arginin sein können.

Die Ausgangsverbindungen für die erwähnten Syntheseverfahren der Benzolsulfonylharnstoffe I werden nach an sich bekannten Methoden hergestellt, wie sie in der Literatur (zum Beispiel in den Standardwerken wie Houben-Weyl, Methoden der Organischen Chemie, Georg Thieme Verlag, Stuttgart; Organic Reactions, John Wiley & Sons, Inc., New York; sowie in den oben angegebenen Patentanmeldungen) beschrieben sind, und zwar unter Reaktionsbedingungen, die für die genannten Umsetzungen bekannt und geeignet sind. Dabei kann man auch von an sich bekannten, hier aber nicht näher erwähnten Varianten Gebrauch machen. Die Ausgangsstoffe können gewünschtenfalls auch in situ gebildet werden, derart, daß man sie aus dem Reaktionsgemisch nicht isoliert, sondern sofort weiter umsetzt.

So kann man geeignet substituierte Carbonsäuren der Formel XIII 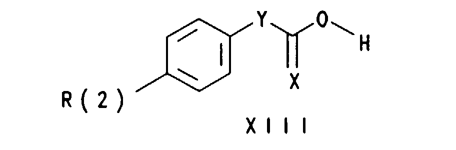 einer Halogensulfonierung unterwerfen und das durch anschließende Ammonolyse erhaltene Sulfonamid XIV 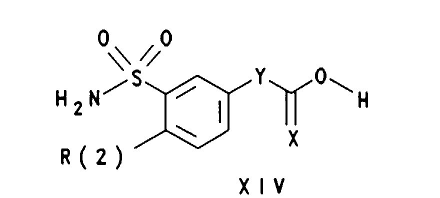 mit einem entsprechenden Amin R(3)R(4)NH nach Aktivierung der Carbonsäuregruppe zum Carbonsäureamid der Formel II 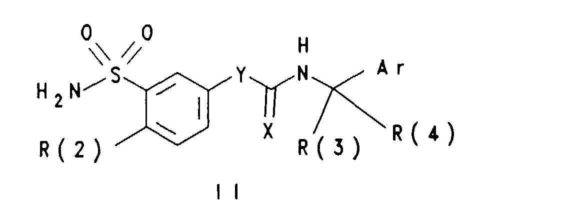 umsetzen.

Als Aktivierungsmethoden eignen sich die Herstellung des Carbonsäurechlorids oder des Esters oder gemischter Carbonsäureanhydride mit Ameisensäurehalogeniden. Desweiteren können die zur Amidbindungsherstellung bekannten Reagenzien wie zum Beispiel Carbonylbisimidazol, Dicyclohexylcarbodiimid und Propanphosphorsäureanhydrid angewandt werden.

Die Sulfonamide XIV werden nach bekannten Methoden hergestellt und zwar unter Reaktionsbedingungen, die für die genannten Umsetzungen bekannt und geeignet sind. Dabei kann man auch von bekannten, hier aber nicht näher erwähnten Varianten Gebrauch machen. Die Synthesen können gewünschtenfalls in einem, zwei oder mehreren Schritten vollzogen werden. Insbesondere sind Verfahren bevorzugt, in denen die Säure XIII durch elektrophile Reagenzien in An- oder Abwesenheit von inerten Lösungsmitteln bei Temperaturen von -10°C bis 120°C, vorzugsweise von 0°C bis 100°C, in aromatische Sulfonsäuren sowie deren Derivate, wie Sulfonsäurehalogenide überführt wird. Beispielsweise können Sulfonierungen mit Schwefelsäuren oder Oleum, Halogensulfonierungen mit Halogensulfonsäuren, Reaktionen mit Sulfurylhalogeniden in Gegenwart von wasserfreien Metallhalogeniden oder Thionylhalogeniden in Gegenwart von wasserfreien Metallhalogeniden mit anschließenden, in bekannter Weise durchgeführten, Oxidationen zu aromatischen Sulfonsäurechloriden, durchgeführt werden. Sind Sulfonsäuren die primären Reaktionsprodukte, so können diese entweder direkt oder durch Behandlung mit tertiären Aminen, wie zum Beispiel Pyridin oder Trialkylaminen oder mit Alkali- oder Erdalkalihydroxiden oder Reagenzien, die diese basischen Verbindung in situ bilden, in bekannter Weise durch Säurehalogenide, wie Phosphortrihalogenide, Phosphorpentahalogenide, Phosphoroxychloride, Thionylhalogenide, Oxalylhalogenide, in Sulfonsäurehalogenide überführt werden. Die Überführung der Sulfonsäurederivate in Sulfonamide erfolgt in literaturbekannter Weise, vorzugsweise werden Sulfonsäurechloride in inerten Lösungsmitteln bei Temperaturen von 0°C bis 100°C mit wäßrigem Ammoniak in Aceton oder THF umgesetzt. Weiterhin kann man aromatische Sulfonamide XIV nach literaturbeschriebenen Verfahren aus den Säuren XIII oder deren Estern durch Umsetzungen mit alkali- oder erdalkalimetallorganischen Reagenzien in inerten Lösungsmitteln und unter Inertgasatmosphäre bei Temperaturen von -100°C bis 50°C, vorzugsweise von -100°C bis 30°C, mit Schwefeldioxid und anschließende thermischer Behandlung mit einem NH₂-Donor, wie etwa Amidosulfonsäure, synthetisieren.

Die erfindungsgemäßen Verbindungen I und ihre physiologisch unbedenklichen Salze können zur Herstellung pharmazeutischer Zubereitungen verwendet werden, insbesondere auf nichtchemischen Weg. Hierbei können sie zusammen mit mindestens einem festen, flüssigen Träger oder Hilfsstoff allein oder in Kombination mit anderen Herz-Kreislauf-aktiven Arzneimitteln, wie etwa Calcium-Antagonisten, NO-Donatoren oder ACE-Hemmern in eine geeignete Dosierungsform gebracht werden. Diese Zubereitungen können als Arzneimittel in der Human- oder Veterinärmedizin verwendet werden. Als Trägerstoffe kommen organische oder anorganische Substanzen in Frage, die sich für die enterale (zum Beispiel orale), parenterale, wie zum Beispiel die intravenöse Applikation, oder topische Anwendungen eignen und mit den neuen Verbindungen nicht reagieren, beispielsweise Wasser, pflanzliche Öle, Benzylalkohole, Polyethylenglykole, Glycerintricacetat, Gelatine, Kohlenhydrate wie Lactose oder Stärke, Magnesiumstearat, Talk, Lanolin, Vaseline. Zur oralen Anwendung dienen insbesondere Tabletten, Dragees, Kapseln, Sirupe, Säfte oder Tropfen, zur rektalen Anwendung Lösungen, vorzugsweise ölige oder wäßrige Lösungen, ferner Suspensionen, Emulsionen oder Implantate, für die topische Anwendung Salben, Cremes, Pasten, Lotionen, Gele, Sprays, Schäume, Aerosole, Lösungen (zum Beispiel in Alkoholen wie Ethanol oder Isopropanol, Acetonitril, DMF, Dimethylacetamid, 1,2-Propandiol oder deren Gemische untereinander oder mit Wasser) oder Puder. Die neuen Verbindungen können auch lyophilisiert und die erhaltenen Lyophilisate zum Beispiel zur Herstellung von Injektionspräparaten verwendet werden. Insbesondere für die topische Anwendung kommen auch liposomale Zubereitungen in Betracht. Die Stabilisierungs- und/oder Netzmittel, Emulgatoren, Salze und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb- und Geschmacks- und/ oder Aromastoffe enthalten. Sie können falls erwünscht auch einen oder mehrere weitere Wirkstoffe enthalten, zum Beispiel ein oder mehrere Vitamine.
Die Dosierungen, die zur Behandlung von Herzrhythmusstörungen mit den Verbindungen I notwendig sind, hängen davon ab, ob akut oder prophylaktisch therapiert wird. Normalerweise kommt man mit einem Dosisbereich von etwa mindestens 0.1 mg, vorzugsweise mindestens 1 mg, bis höchstens 100 mg, vorzugsweise 10 mg, pro kg und Tag aus, wenn Prophylaxe betrieben wird. Die Dosis kann dabei als orale oder parenterale Einzeldosis oder aber in bis zu vier Einzeldosen aufgeteilt werden. Werden akute Fälle von Herzrhythmusstörungen behandelt, beispielsweise auf einer Intensivstation, kann die parenterale Verabreichung vorteilhaft sein. Eine bevorzugte Dosisbereich in kritischen Situationen kann dann 10 bis 100 mg betragen und beispielsweise als intravenöse Dauerinfusion verabreicht werden.

Erfindungsgemäß können außer den in den Ausführungsbeispielen beschriebenen Verbindungen auch die in der folgenden Tabelle zusammengestellten Verbindungen I erhalten werden:
1) N-5-(1-Phenylethyl)aminocarbonylmethyl-2-propoxyphenylsulfonyl-N'-methylthioharnstoff
2) N-5-(1-(2-Furyl)-ethyl)aminocarbonylmethyl-2-methoxyphenylsulfonyl-N'-methylthioharnstoff
3) N-5-(1-(3-Furyl)-ethyl)aminocarbonylmethyl-2-methoxyphenylsulfonyl-N'-methylthioharnstoff
4) N-5-(1-(2-Thienyl)-ethyl)aminocarbonylmethyl-2-methoxyphenylsulfonyl-N'-methylthioharnstoff
5) N-5-(1-(3-Thienyl)-ethyl)aminocarbonylmethyl-2-methoxyphenylsulfonyl-N'-methylthioharnstoff
6) N-5-(1-(2-Pyrrolyl)-ethyl)aminocarbonylmethyl-2-methoxyphenylsulfonyl-N'-methylthioharnstoff
7) N-5-(1-(3-Pyrrolyl)-ethyl)aminocarbonylmethyl-2-methoxyphenylsulfonyl-N'-methylthioharnstoff
8) N-5-(1-(2-Thiazolyl)-ethyl)aminocarbonylmethyl-2-methoxyphenylsulfonyl-N'-methylthioharnstoff
9) N-5-(1-(3-Thiazolyl)-ethyl)aminocarbonylmethyl-2-methoxyphenylsulfonyl-N'-methylthioharnstoff
10) N-5-(1-(2-Furyl)-ethyl)aminocarbonylmethyl-2-methoxyphenylsulfonyl-N'-methylharnstoff
11) N-5-(1-(3-Furyl)-ethyl)aminocarbonylmethyl-2-methoxyphenylsulfonyl-N'-methylharnstoff
12) N-5-(1-(2-Thienyl)-ethyl)aminocarbonylmethyl-2-methoxyphenylsulfonyl-N'-methylharnstoff
13) N-5-( 1 -(3-Thienyl)-ethyl)aminocarbonylmethyl-2-methoxyphenylsulfonyl-N'-methylharnstoff
14) N-5-(1-(2-Pyrrolyl)-ethyl)aminocarbonylmethyl-2-methoxyphenylsulfonyl-N'-methylharnstoff
15) N-5-(1-(3-Pyrrolyl)-ethyl)aminocarbonylmethyl-2-methoxyphenylsulfonyl-N'-methylharnstoff
16) N-5-(1-(2-Thiazolyl)-ethyl)aminocarbonylmethyl-2-methoxyphenylsulfonyl-N'-methylharnstoff
17) N-5-( 1 -(2-Furyl)-ethyl)aminocarbonylmethyl-2-methylphenylsulfonyl-N'-methylthioharnstoff
18) N-5-(1-(3-Furyl)-ethyl)aminocarbonylmethyl-2-methylphenylsulfonyl-N'-methylthioharnstoff
19) N-5-(1-(2-Thienyl)-ethyl)aminocarbonylmethyl-2-methylphenylsulfonyl-N'-methylthioharnstoff
20) N-5-(1-(3-Thienyl)-ethyl)aminocarbonylmethyl-2-methylphenylsulfonyl-N'-methylthioharnstoff
21) N-5-(1-(2-Pyrrolyl)-ethyl)aminocarbonylmethyl-2-methylphenylsulfonyl-N'-methylthioharnstoff
22) N-5-(1-(3-Pyrrolyl)-ethyl)aminocarbonylmethyl-2-methylphenylsulfonyl-N'-methylthioharnstoff
23) N-5-(1-(2-Thiazolyl)-ethyl)aminocarbonylmethyl-2-methylphenylsulfonyl-N'-methylthioharnstoff
24) N-5-(1-(3-Thiazolyl)-ethyl)aminocarbonylmethyl-2-methylphenylsulfonyl-N'-methylthioharnstoff
25) N-5-(1-(2-Furyl)-ethyl)aminocarbonylmethyl-2-methylphenylsulfonyl-N'-methylharnstoff
26) N-5-(1-(3-Furyl)-ethyl)aminocarbonylmethyl-2-methylphenylsulfonyl-N'-methylharnstoff
27) N-5-(1-(2-Thienyl)-ethyl)aminocarbonylmethyl-2-methylphenylsulfonyl-N'-methylharnstoff
28) N-5-(1-(3-Thienyl)-ethyl)aminocarbonylmethyl-2-methylphenylsulfonyl-N'-methylharnstoff
29) N-5-(1-(2-Pyrrolyl)-ethyl)aminocarbonylmethyl-2-methylphenylsulfonyl-N'-methylharnstoff
30) N-5-(1-(3-Pyrrolyl)-ethyl)aminocarbonylmethyl-2-methylphenylsulfonyl-N'-methylharnstoff
31) N-5-(1-(2-Thiazolyl)-ethyl)aminocarbonylmethyl-2-methylphenylsulfonyl-N'-methylharnstoff
32) N-5-(1-(3-Thiazolyl)-ethyl)aminocarbonylmethyl-2-methylthiophenylsulfonyl-N'-methylharnstoff
33) N-5-(1-(2-Furyl)-ethyl)aminocarbonylmethyl-2-methylthiophenylsulfonyl-N'-methylthioharnstoff
34) N-5-(1-(3-Furyl)-ethyl)aminocarbonylmethyl-2-methylthiophenylsulfonyl-N'-methylthioharnstoff
35) N-5-(1-(2-Thienyl)-ethyl)aminocarbonylmethyl-2-methylthiophenylsulfonyl-N'-methylthioharnstoff
36) N-5-(1-(3-Thienyl)-ethyl)aminocarbonylmethyl-2-methylthiophenylsulfonyl-N'-methylthioharnstoff
37) N-5-(1-(2-Pyrrolyl)-ethyl)aminocarbonylmethyl-2-methylthiophenylsulfonyl-N'-methylthioharnstoff
38) N-5-(1-(3-Pyrrolyl)-ethyl)aminocarbonylmethyl-2-methylthiophenylsulfonyl-N'-methylthioharnstoff
39) N-5-(1-(2-Thiazolyl)-ethyl)aminocarbonylmethyl-2-methylthiophenylsulfonyl-N'-methylthioharnstoff
40) N-5-(1-(3-Thiazolyl)-ethyl)aminocarbonylmethyl-2-methylthiophenylsulfonyl-N'-methylthioharnstoff
41) N-5-(1-(2-Furyl)-ethyl)aminocarbonylmethyl-2-methylthiophenylsulfonyl-N'-methylharnstoff
42) N-5-(1-(3-Furyl)-ethyl)aminocarbonylmethyl-2-methylthiophenylsulfonyl-N'-methylharnstoff
43) N-5-(1-(2-Thienyl)-ethyl)aminocarbonylmethyl-2-methylthiophenylsulfonyl-N'-methylharnstoff
44) N-5-(1-(3-Thienyl)-ethyl)aminocarbonylmethyl-2-methylthiophenylsulfonyl-N'-methylharnstoff
45) N-5-(1-(2-Pyrrolyl)-ethyl)aminocarbonylmethyl-2-methylthiophenylsulfonyl-N'-methylharnstoff
46) N-5-(1-(3-Pyrrolyl)-ethyl)aminocarbonylmethyl-2-methylthiophenylsulfonyl-N'-methylharnstoff
47) N-5-(1-(2-Thiazolyl)-ethyl)aminocarbonylmethyl-2-methylthiophenylsulfonyl-N'-methylharnstoff
48) N-5-(1-(2-Furyl)-ethyl)aminocarbonylmethyl-2-ethoxyphenylsulfonyl-N'-methylthioharnstoff
49) N-5-(1-(3-Furyl)-ethyl)aminocarbonylmethyl-2-ethoxyphenylsulfonyl-N'-methylthioharnstoff
50) N-5-(1-(2-Thienyl)-ethyl)aminocarbonylmethyl-2-ethoxyphenylsulfonyl-N'-methylthioharnstoff
51) N-5-(1-(3-Thienyl)-ethyl)aminocarbonylmethyl-2-ethoxyphenylsulfonyl-N'-methylthioharnstoff
52) N-5-(1-(2-Pyrrolyl)-ethyl)aminocarbonylmethyl-2-ethoxyphenylsulfonyl-N'-methylthioharnstoff
53) N-5-(1-(3-Pyrrolyl)-ethyl)aminocarbonylmethyl-2-ethoxyphenylsulfonyl-N'-methylthioharnstoff
54) N-5-(1-(2-Thiazolyl)-ethyl)aminocarbonylmethyl-2-ethoxyphenylsulfonyl-N'-methylthioharnstoff
55) N-5-(1-(3-Thiazolyl)-ethyl)aminocarbonylmethyl-2-ethoxyphenylsulfonyl-N'-methylthioharnstoff
56) N-5-(1-(2-Furyl)-ethyl)aminocarbonylmethyl-2-ethoxyphenylsulfonyl-N'-methylharnstoff
57) N-5-(1-(3-Furyl)-ethyl)aminocarbonylmethyl-2-ethoxyphenylsulfonyl-N'-methylharnstoff
58) N-5-(1-(2-Thienyl)-ethyl)aminocarbonylmethyl-2-ethoxyphenylsulfonyl-N'-methylharnstoff
59) N-5-(1-(3-Thienyl)-ethyl)aminocarbonylmethyl-2-ethoxyphenylsulfonyl-N'-methylharnstoff
60) N-5-(1-(2-Pyrrolyl)-ethyl)aminocarbonylmethyl-2-ethoxyphenylsulfonyl-N'-methylharnstoff
61) N-5-(1-(3-Pyrrolyl)-ethyl)aminocarbonylmethyl-2-ethoxyphenylsulfonyl-N'-methylharnstoff
62) N-5-(1-(2-Thiazolyl)-ethyl)aminocarbonylmethyl-2-ethoxyphenylsulfonyl-N'-methylharnstoff
63) N-5-(1-(2-Furyl)-ethyl)aminocarbonylmethyl-2-propoxyphenylsulfonyl-N'-methylthioharnstoff
64) N-5-(1-(3-Furyl)-ethyl)aminocarbonylmethyl-2-propoxyphenylsulfonyl-N'-methylthioharnstoff
65) N-5-(1-(2-Thienyl)-ethyl)aminocarbonylmethyl-2-propoxyphenylsulfonyl-N'-methylthioharnstoff
66) N-5-(1-(3-Thienyl)-ethyl)aminocarbonylmethyl-2-propoxyphenylsulfonyl-N'-methylthioharnstoff
67) N-5-(1-(2-Pyrrolyl)-ethyl)aminocarbonylmethyl-2-propoxyphenylsulfonyl-N'-methylthioharnstoff
68) N-5-(1-(3-Pyrrolyl)-ethyl)aminocarbonylmethyl-2-propoxyphenylsulfonyl-N'-methylthioharnstoff
69) N-5-(1-(2-Thiazolyl)-ethyl)aminocarbonylmethyl-2-propoxyphenylsulfonyl-N'-methylthioharnstoff
70) N-5-(1-(3-Thiazolyl)-ethyl)aminocarbonylmethyl-2-propoxyphenylsulfonyl-N'-methylthioharnstoff
71) N-5-(1-(2-Furyl)-ethyl)aminocarbonylmethyl-2-propoxyphenylsulfonyl-N'-methylharnstoff
72) N-5-(1-(3-Furyl)-ethyl)aminocarbonylmethyl-2-propoxyphenylsulfonyl-N'-methylharnstoff
73) N-5-(1-(2-Thienyl)-ethyl)aminocarbonylmethyl-2-propoxyphenylsulfonyl-N'-methylharnstoff
74) N-5-(1-(3-Thienyl)-ethyl)aminocarbonylmethyl-2-propoxyphenylsulfonyl-N'-methylharnstoff
75) N-5-(1-(2-Pyrrolyl)-ethyl)aminocarbonylmethyl-2-propoxyphenylsulfonyl-N'-methylharnstoff
76) N-5-(1-(3-Pyrrolyl)-ethyl)aminocarbonylmethyl-2-propoxyphenylsulfonyl-N'-methylharnstoff
77) N-5-(1-(2-Thiazolyl)-ethyl)aminocarbonylmethyl-2-propoxyphenylsulfonyl-N'-methylharnstoff
78) N-5-(1-Phenylpropyl)aminocarbonylmethyl-2-propoxyphenylsulfonyl-N'-methylthioharnstoff
79) N-5-(1-(2-Furyl)-propyl)aminocarbonylmethyl-2-methoxyphenylsulfonyl-N'-methylthioharnstoff
80) N-5-(1-(3-Furyl)-propyl)aminocarbonylmethyl-2-methoxyphenylsulfonyl-N'-methylthioharnstoff
81) N-5-(1-(2-Thienyl)-propyl)aminocarbonylmethyl-2-methoxyphenylsulfonyl-N'-methylthioharnstoff
82) N-5-(1-(3-Thienyl)-propyl)aminocarbonylmethyl-2-methoxyphenylsulfonyl-N'-methylthioharnstoff
83) N-5-(1-(2-Pyrrolyl)-propyl)aminocarbonylmethyl-2-methoxyphenylsulfonyl-N'-methylthioharnstoff
84) N-5-(1-(3-Pyrrolyl)-propyl)aminocarbonylmethyl-2-methoxyphenylsulfonyl-N'-methylthioharnstoff
85) N-5-(1-(2-Thiazolyl)-propyl)aminocarbonylmethyl-2-methoxyphenylsulfonyl-N'-methylthioharnstoff
86) N-5-(1-(3-Thiazolyl)-propyl)aminocarbonylmethyl-2-methoxyphenylsulfonyl-N'-methylthioharnstoff
87) N-5-(1-(2-Furyl)-propyl)aminocarbonylmethyl-2-methoxyphenylsulfonyl-N'-methylharnstoff
88) N-5-(1-(3-Furyl)-propyl)aminocarbonylmethyl-2-methoxyphenylsulfonyl-N'-methylharnstoff
89) N-5-(1-(2-Thienyl)-propyl)aminocarbonylmethyl-2-methoxyphenylsulfonyl-N'-methylharnstoff
90) N-5-(1-(3-Thienyl)-propyl)aminocarbonylmethyl-2-methoxyphenylsulfonyl-N'-methylharnstoff
91) N-5-(1-(2-Pyrrolyl)-propyl)aminocarbonylmethyl-2-methoxyphenylsulfonyl-N'-methylharnstoff
92) N-5-(1-(3-Pyrrolyl)-propyl)aminocarbonylmethyl-2-methoxyphenylsulfonyl-N'-methylharnstoff
93) N-5-(1-(2-Thiazolyl)-propyl)aminocarbonylmethyl-2-methoxyphenylsulfonyl-N'-methylharnstoff
94) N-5-(1-(2-Furyl)-propyl)aminocarbonylmethyl-2-methylphenylsulfonyl-N'-methylthioharnstoff
95) N-5-(1-(3-Furyl)-propyl)aminocarbonylmethyl-2-methylphenylsulfonyl-N'-methylthioharnstoff
96) N-5-(1-(2-Thienyl)-propyl)aminocarbonylmethyl-2-methylphenylsulfonyl-N'-methylthioharnstoff
97) N-5-(1-(3-Thienyl)-propyl)aminocarbonylmethyl-2-methylphenylsulfonyl-N'-methylthioharnstoff
98) N-5-(1-(2-Pyrrolyl)-propyl)aminocarbonylmethyl-2-methylphenylsulfonyl-N'-methylthioharnstoff
99) N-5-(1-(3-Pyrrolyl)-propyl)aminocarbonylmethyl-2-methylphenylsulfonyl-N'-methylthioharnstoff
100) N-5-(1-(2-Thiazolyl)-propyl)aminocarbonylmethyl-2-methylphenylsulfonyl-N'-methylthioharnstoff
101) N-5-(1-(3-Thiazolyl)-propyl)aminocarbonylmethyl-2-methylphenylsulfonyl-N'-methylthioharnstoff
102) N-5-(1-(2-Furyl)-propyl)aminocarbonylmethyl-2-methylphenylsulfonyl-N'-methylharnstoff
103) N-5-(1-(3-Furyl)-propyl)aminocarbonylmethyl-2-methylphenylsulfonyl-N'-methylharnstoff
104) N-5-(1-(2-Thienyl)-propyl)aminocarbonylmethyl-2-methylphenylsulfonyl-N'-methylharnstoff
105) N-5-(1-(3-Thienyl)-propyl)aminocarbonylmethyl-2-methylphenylsulfonyl-N'-methylharnstoff
106) N-5-(1-(2-Pyrrolyl)-propyl)aminocarbonylmethyl-2-methylphenylsulfonyl-N'-methylharnstoff
107) N-5-(1-(3-Pyrrolyl)-propyl)aminocarbonylmethyl-2-methylphenylsulfonyl-N'-methylharnstoff
108) N-5-(1-(2-Thiazolyl)-propyl)aminocarbonylmethyl-2-methylphenylsulfonyl-N'-methylharnstoff
109) N-5-(1-(3-Thiazolyl)-propyl)aminocarbonylmethyl-2-methylthiophenylsulfonyl-N'-methylharnstoff
110) N-5-(1-(2-Furyl)-propyl)aminocarbonylmethyl-2-methylthiophenylsulfonyl-N'-methylthioharnstoff
111) N-5-(1-(3-Furyl)-propyl)aminocarbonylmethyl-2-methylthiophenylsulfonyl-N'-methylthioharnstoff
112) N-5-(1-(2-Thienyl)-propyl)aminocarbonylmethyl-2-methylthiophenylsulfonyl-N'-methylthioharnstoff
113) N-5-(1-(3-Thienyl)-propyl)aminocarbonylmethyl-2-methylthiophenylsulfonyl-N'-methylthioharnstoff
114) N-5-(1-(2-Pyrrolyl)-propyl)aminocarbonylmethyl-2-methylthiophenylsulfonyl-N'-methylthioharnstoff
115) N-5-(1-(3-Pyrrolyl)-propyl)aminocarbonylmethyl-2-methylthiophenylsulfonyl-N'-methylthioharnstoff
116) N-5-(1-(2-Thiazolyl)-propyl)aminocarbonylmethyl-2-methylthiophenylsulfonyl-N'-methylthioharnstoff
117) N-5-(1-(3-Thiazolyl)-propyl)aminocarbonylmethyl-2-methylthiophenylsulfonyl-N'-methylthioharnstoff
118) N-5-(1-(2-Furyl)-propyl)aminocarbonylmethyl-2-methylthiophenylsulfonyl-N'-methylharnstoff
119) N-5-(1-(3-Furyl)-propyl)aminocarbonylmethyl-2-methylthiophenylsulfonyl-N'-methylharnstoff
120) N-5-(1-(2-Thienyl)-propyl)aminocarbonylmethyl-2-methylthiophenylsulfonyl-N'-methylharnstoff
121) N-5-(1-(3-Thienyl)-propyl)aminocarbonylmethyl-2-methylthiophenylsulfonyl-N'-methylharnstoff
122) N-5-(1-(2-Pyrrolyl)-propyl)aminocarbonylmethyl-2-methylthiophenylsulfonyl-N'-methylharnstoff
123) N-5-(1-(3-Pyrrolyl)-propyl)aminocarbonylmethyl-2-methylthiophenylsulfonyl-N'-methylharnstoff
124) N-5-(1-(2-Thiazolyl)-propyl)aminocarbonylmethyl-2-methylthiophenylsulfonyl-N'-methylharnstoff
125) N-5-(1-(2-Furyl)-propyl)aminocarbonylmethyl-2-ethoxyphenylsulfonyl-N'-methylthioharnstoff
126) N-5-(1-(3-Furyl)-propyl)aminocarbonylmethyl-2-ethoxyphenylsulfonyl-N'-methylthioharnstoff
127) N-5-(1-(2-Thienyl)-propyl)aminocarbonylmethyl-2-ethoxyphenylsulfonyl-N'-methylthioharnstoff
128) N-5-(1-(3-Thienyl)-propyl)aminocarbonylmethyl-2-ethoxyphenylsulfonyl-N'-methylthioharnstoff
129) N-5-(1-(2-Pyrrolyl)-propyl)aminocarbonylmethyl-2-ethoxyphenylsulfonyl-N'-methylthioharnstoff
130) N-5-(1-(3-Pyrrolyl)-propyl)aminocarbonylmethyl-2-ethoxyphenylsulfonyl-N'-methylthioharnstoff
131) N-5-(1-(2-Thiazolyl)-propyl)aminocarbonylmethyl-2-ethoxyphenylsulfonyl-N'-methylthioharnstoff
132) N-5-(1-(3-Thiazolyl)-propyl)aminocarbonylmethyl-2-ethoxyphenylsulfonyl-N'-methylthioharnstoff
133) N-5-(1-(2-Furyl)-propyl)aminocarbonylmethyl-2-ethoxyphenylsulfonyl-N'-methylharnstoff
134) N-5-(1-(3-Furyl)-propyl)aminocarbonylmethyl-2-ethoxyphenylsulfonyl-N'-methylharnstoff
135) N-5-(1-(2-Thienyl)-propyl)aminocarbonylmethyl-2-ethoxyphenylsulfonyl-N'-methylharnstoff
136) N-5-(1-(3-Thienyl)-propyl)aminocarbonylmethyl-2-ethoxyphenylsulfonyl-N'-methylharnstoff
137) N-5-(1-(2-Pyrrolyl)-propyl)aminocarbonylmethyl-2-ethoxyphenylsulfonyl-N'-methylharnstoff
138) N-5-(1-(3-Pyrrolyl)-propyl)aminocarbonylmethyl-2-ethoxyphenylsulfonyl-N'-methylharnstoff
139) N-5-(1-(2-Thiazolyl)-propyl)aminocarbonylmethyl-2-ethoxyphenylsulfonyl-N'-methylharnstoff
140) N-5-(1-(2-Furyl)-propyl)aminocarbonylmethyl-2-propoxyphenylsulfonyl-N'-methylthioharnstoff
141) N-5-(1-(3-Furyl)-propyl)aminocarbonylmethyl-2-propoxyphenylsulfonyl-N'-methylthioharnstoff
142) N-5-(1-(2-Thienyl)-propyl)aminocarbonylmethyl-2-propoxyphenylsulfonyl-N'-methylthioharnstoff
143) N-5-(1-(3-Thienyl)-propyl)aminocarbonylmethyl-2-propoxyphenylsulfonyl-N'-methylthioharnstoff
144) N-5-(1-(2-Pyrrolyl)-propyl)aminocarbonylmethyl-2-propoxyphenylsulfonyl-N'-methylthioharnstoff
145) N-5-(1-(3-Pyrrolyl)-propyl)aminocarbonylmethyl-2-propoxyphenylsulfonyl-N'-methylthioharnstoff
146) N-5-(1-(2-Thiazolyl)-propyl)aminocarbonylmethyl-2-propoxyphenylsulfonyl-N'-methylthioharnstoff
147) N-5-(1-(3-Thiazolyl)-propyl)aminocarbonylmethyl-2-propoxyphenylsulfonyl-N'-methylthioharnstoff
148) N-5-(1-(2-Furyl)-propyl)aminocarbonylmethyl-2-propoxyphenylsulfonyl-N'-methylharnstoff
149) N-5-(1-(3-Furyl)-propyl)aminocarbonylmethyl-2-propoxyphenylsulfonyl-N'-methylharnstoff
150) N-5-(1-(2-Thienyl)-propyl)aminocarbonylmethyl-2-propoxyphenylsulfonyl-N'-methylharnstoff
151) N-5-(1-(3-Thienyl)-propyl)aminocarbonylmethyl-2-propoxyphenylsulfonyl-N'-methylharnstoff
152) N-5-(1-(2-Pyrrolyl)-propyl)aminocarbonylmethyl-2-propoxyphenylsulfonyl-N'-methylharnstoff
153) N-5-(1-(3-Pyrrolyl)-propyl)aminocarbonylmethyl-2-propoxyphenylsulfonyl-N'-methylharnstoff
154) N-5-(1-(2-Thiazolyl)-propyl)aminocarbonylmethyl-2-propoxyphenylsulfonyl-N'-methylharnstoff
155) N-5-(1-Phenyl-1-methylethyl)aminocarbonylmethyl-2-propoxyphenylsulfonyl-N'-methylthioharnstoff
156) N-5-(1-(2-Furyl)-1-methylethyl)aminocarbonylmethyl-2-methoxyphenylsulfonyl-N'-methylthioharnstoff
157) N-5-(1-(3-Furyl)-1-methylethyl)aminocarbonylmethyl-2-methoxyphenylsulfonyl-N'-methylthioharnstoff
158) N-5-(1-(2-Thienyl)-1-methylethyl)aminocarbonylmethyl-2-methoxyphenylsulfonyl-N'-methylthioharnstoff
159) N-5-(1-(3-Thienyl)-1-methylethyl)aminocarbonylmethyl-2-methoxyphenylsulfonyl-N'-methylthioharnstoff
160) N-5-(1-(2-Pyrrolyl)-1-methylethyl)aminocarbonylmethyl-2-methoxyphenylsulfonyl-N'-methylthioharnstoff
161) N-5-(1-(3-Pyrrolyl)-1-methylethyl)aminocarbonylmethyl-2-methoxyphenylsulfonyl-N'-methylthioharnstoff
162) N-5-(1-(2-Thiazolyl)-1-methylethyl)aminocarbonylmethyl-2-methoxyphenylsulfonyl-N'-methylthioharnstoff
163) N-5-(1-(3-Thiazolyl)-1-methylethyl)aminocarbonylmethyl-2-methoxyphenylsulfonyl-N'-methylthioharnstoff
164) N-5-(1-(2-Furyl)-1-methylethyl)aminocarbonylmethyl-2-methoxyphenylsulfonyl-N'-methylharnstoff
165) N-5-(1-(3-Furyl)-1-methylethyl)aminocarbonylmethyl-2-methoxyphenylsulfonyl-N'-methylharnstoff
166) N-5-(1-(2-Thienyl)-1-methylethyl)aminocarbonylmethyl-2-methoxyphenylsulfonyl-N'-methylharnstoff
167) N-5-(1-(3-Thienyl)-1-methylethyl)aminocarbonylmethyl-2-methoxyphenylsulfonyl-N'-methylharnstoff
168) N-5-(1-(2-Pyrrolyl)-1-methylethyl)aminocarbonylmethyl-2-methoxyphenylsulfonyl-N'-methylharnstoff
169) N-5-(1-(3-Pyrrolyl)-1-methylethyl)aminocarbonylmethyl-2-methoxyphenylsulfonyl-N'-methylharnstoff
170) N-5-(1-(2-Thiazolyl)-1-methylethyl)aminocarbonylmethyl-2-methoxyphenylsulfonyl-N'-methylharnstoff
171) N-5-(1-(2-Furyl)-1-methylethyl)aminocarbonylmethyl-2-methylphenylsulfonyl-N'-methylthioharnstoff
172) N-5-(1-(3-Furyl)-1-methylethyl)aminocarbonylmethyl-2-methylphenylsulfonyl-N'-methylthioharnstoff
173) N-5-(1-(2-Thienyl)-1-methylethyl)aminocarbonylmethyl-2-methylphenylsulfonyl-N'-methylthioharnstoff
174) N-5-(1-(3-Thienyl)-1-methylethyl)aminocarbonylmethyl-2-methylphenylsulfonyl-N'-methylthioharnstoff
175) N-5-(1-(2-Pyrrolyl)-1-methylethyl)aminocarbonylmethyl-2-methylphenylsulfonyl-N'-methylthioharnstoff
176) N-5-(1-(3-Pyrrolyl)-1-methylethyl)aminocarbonylmethyl-2-methylphenylsulfonyl-N'-methylthioharnstoff
177) N-5-(1-(2-Thiazolyl)-1-methylethyl)aminocarbonylmethyl-2-methylphenylsulfonyl-N'-methylthioharnstoff
178) N-5-(1-(3-Thiazolyl)-1-methylethyl)aminocarbonylmethyl-2-methylphenylsulfonyl-N'-methylthioharnstoff
179) N-5-(1-(2-Furyl)-1-methylethyl)aminocarbonylmethyl-2-methylphenylsulfonyl-N'-methylharnstoff
180) N-5-(1-(3-Furyl)-1-methylethyl)aminocarbonylmethyl-2-methylphenylsulfonyl-N'-methylharnstoff
181) N-5-(1-(2-Thienyl)-1-methylethyl)aminocarbonylmethyl-2-methylphenylsulfonyl-N'-methylharnstoff
182) N-5-(1-(3-Thienyl)-1-methylethyl)aminocarbonylmethyl-2-methylphenylsulfonyl-N'-methylharnstoff
183) N-5-(1-(2-Pyrrolyl)-1-methylethyl)aminocarbonylmethyl-2-methylphenylsulfonyl-N'-methylharnstoff
184) N-5-(1-(3-Pyrrolyl)-1-methylethyl)aminocarbonylmethyl-2-methylphenylsulfonyl-N'-methylharnstoff
185) N-5-(1-(2-Thiazolyl)-1-methylethyl)aminocarbonylmethyl-2-methylphenylsulfonyl-N'-methylharnstoff
186) N-5-(1-(3-Thiazolyl)-1-methylethyl)aminocarbonylmethyl-2-methylthiophenylsulfonyl-N'-methylharnstoff
187) N-5-(1-(2-Furyl)-1-methylethyl)aminocarbonylmethyl-2-methylthiophenylsulfonyl-N'-methylthioharnstoff
188) N-5-(1-(3-Furyl)-1-methylethyl)aminocarbonylmethyl-2-methylthiophenylsulfonyl-N'-methylthioharnstoff
189) N-5-(1-(2-Thienyl)-1-methylethyl)aminocarbonylmethyl-2-methylthiophenylsulfonyl-N'-methylthioharnstoff
190) N-5-(1-(3-Thienyl)-1-methylethyl)aminocarbonylmethyl-2-methylthiophenylsulfonyl-N'-methylthioharnstoff
191) N-5-(1-(2-Pyrrolyl)-1-methylethyl)aminocarbonylmethyl-2-methylthiophenylsulfonyl-N'-methylthioharnstoff
192) N-5-(1-(3-Pyrrolyl)-1-methylethyl)aminocarbonylmethyl-2-methylthiophenylsulfonyl-N'-methylthioharnstoff
193) N-5-(1-(2-Thiazolyl)-1-methylethyl)aminocarbonylmethyl-2-methylthiophenylsulfonyl-N'-methylthioharnstoff
194) N-5-(1-(3-Thiazolyl)-1-methylethyl)aminocarbonylmethyl-2-methylthiophenylsulfonyl-N'-methylthioharnstoff
195) N-5-(1-(2-Furyl)-1-methylethyl)aminocarbonylmethyl-2-methylthiophenylsulfonyl-N'-methylharnstoff
196) N-5-(1-(3-Furyl)-1-methylethyl)aminocarbonylmethyl-2-methylthiophenylsulfonyl-N'-methylharnstoff
197) N-5-(1-(2-Thienyl)-1-methylethyl)aminocarbonylmethyl-2-methylthiophenylsulfonyl-N'-methylharnstoff
198) N-5-(1-(3-Thienyl)-1-methylethyl)aminocarbonylmethyl-2-methylthiophenylsulfonyl-N'-methylharnstoff
199) N-5-(1-(2-Pyrrolyl)-1-methylethyl)aminocarbonylmethyl-2-methylthiophenylsulfonyl-N'-methylharnstoff
200) N-5-(1-(3-Pyrrolyl)-1-methylethyl)aminocarbonylmethyl-2-methylthiophenylsulfonyl-N'-methylharnstoff
201) N-5-(1-(2-Thiazolyl)-1-methylethyl)aminocarbonylmethyl-2-methylthiophenylsulfonyl-N'-methylharnstoff
202) N-5-(1-(2-Furyl)-1-methylethyl)aminocarbonylmethyl-2-ethoxyphenylsulfonyl-N'-methylthioharnstoff
203) N-5-(1-(3-Furyl)-1-methylethyl)aminocarbonylmethyl-2-ethoxyphenylsulfonyl-N'-methylthioharnstoff
204) N-5-(1-(2-Thienyl)-1-methylethyl)aminocarbonylmethyl-2-ethoxyphenylsulfonyl-N'-methylthioharnstoff
205) N-5-(1-(3-Thienyl)-1-methylethyl)aminocarbonylmethyl-2-ethoxyphenylsulfonyl-N'-methylthioharnstoff
206) N-5-(1-(2-Pyrrolyl)-1-methylethyl)aminocarbonylmethyl-2-ethoxyphenylsulfonyl-N'-methylthioharnstoff
207) N-5-(1-(3-Pyrrolyl)-1-methylethyl)aminocarbonylmethyl-2-ethoxyphenylsulfonyl-N'-methylthioharnstoff
208) N-5-(1-(2-Thiazolyl)-1-methylethyl)aminocarbonylmethyl-2-ethoxyphenylsulfonyl-N'-methylthioharnstoff
209) N-5-(1-(3-Thiazolyl)-1-methylethyl)aminocarbonylmethyl-2-ethoxyphenylsulfonyl-N'-methylthioharnstoff
210) N-5-(1-(2-Furyl)-1-methylethyl)aminocarbonylmethyl-2-ethoxyphenylsulfonyl-N'-methylharnstoff
211) N-5-(1-(3-Furyl)-1-methylethyl)aminocarbonylmethyl-2-ethoxyphenylsulfonyl-N'-methylharnstoff
212) N-5-(1-(2-Thienyl)-1-methylethyl)aminocarbonylmethyl-2-ethoxyphenylsulfonyl-N'-methylharnstoff
213) N-5-(1-(3-Thienyl)-1-methylethyl)aminocarbonylmethyl-2-ethoxyphenylsulfonyl-N'-methylharnstoff
214) N-5-(1-(2-Pyrrolyl)-1-methylethyl)aminocarbonylmethyl-2-ethoxyphenylsulfonyl-N'-methylharnstoff
21 5) N-5-(1-(3-Pyrrolyl)-1-methylethyl)aminocarbonylmethyl-2-ethoxyphenylsulfonyl-N'-methylharnstoff
216) N-5-(1-(2-Thiazolyl)-1-methylethyl)aminocarbonylmethyl-2-ethoxyphenylsulfonyl-N'-methylharnstoff
217) N-5-(1-(2-Furyl)-1-methylethyl)aminocarbonylmethyl-2-propoxyphenylsulfonyl-N'-methylthioharnstoff
218) N-5-(1-(3-Furyl)-1-methylethyl)aminocarbonylmethyl-2-propoxyphenylsulfonyl-N'-methylthioharnstoff
219) N-5-(1-(2-Thienyl)-1-methylethyl)aminocarbonylmethyl-2-propoxyphenylsulfonyl-N'-methylthioharnstoff
220) N-5-(1-(3-Thienyl)-1-methylethyl)aminocarbonylmethyl-2-propoxyphenylsulfonyl-N'-methylthioharnstoff
221) N-5-(1-(2-Pyrrolyl)-1-methylethyl)aminocarbonylmethyl-2-propoxyphenylsulfonyl-N'-methylthioharnstoff
222) N-5-(1-(3-Pyrrolyl)-1-methylethyl)aminocarbonylmethyl-2-propoxyphenylsulfonyl-N'-methylthioharnstoff
223) -5-(1-(2-Thiazolyl)-1-methylethyl)aminocarbonylmethyl-2-propoxyphenylsulfonyl-N'-methylthioharnstoff
224) N-5-(1-(3-Thiazolyl)-1-methylethyl)aminocarbonylmethyl-2-propoxyphenylsulfonyl-N'-methylthioharnstoff
225) N-5-(1-(2-Furyl)-1-methylethyl)aminocarbonylmethyl-2-propoxyphenylsulfonyl-N'-methylharnstoff
226) N-5-(1-(3-Furyl)-1-methylethyl)aminocarbonylmethyl-2-propoxyphenylsulfonyl-N'-methylharnstoff
227) N-5-(1-(2-Thienyl)-1-methylethyl)aminocarbonylmethyl-2-propoxyphenylsulfonyl-N'-methylharnstoff
228) N-5-(1-(3-Thienyl)-1-methylethyl)aminocarbonylmethyl-2-propoxyphenylsulfonyl-N'-methylharnstoff
229) N-5-(1-(2-Pyrrolyl)-1-methylethyl)aminocarbonylmethyl-2-propoxyphenylsulfonyl-N'-methylharnstoff
230) N-5-(1-(3-Pyrrolyl)-1-methylethyl)aminocarbonylmethyl-2-propoxyphenylsulfonyl-N'-methylharnstoff
231) N-5-(1-(2-Thiazolyl)-1-methylethyl)aminocarbonylmethyl-2-propoxyphenylsulfonyl-N'-methylharnstoff

### Herstellung der Ausgangsmaterialien

### Herstellung von 3-Sulfamoylphenylalkancarbonsäuren

Die 4-substituierten Phenylalkancarbonsäuren werden unter Rühren portionsweise zu einem Überschuß an Chlorsulfonsäure gegeben. Man rührt 30 Minuten bei Raumtemperatur, gießt anschließend auf Eis und saugt das entstandene Sulfonsäurechlorid ab. Dieses wird in Ammoniaklösung gelöst, 30 Minuten bei Raumtemperatur gerührt, und die Lösung wird mit 2N Salzsäure neutralisiert. Das erhaltene Produkt wird abgesaugt.

Nach dieser Methode hergestellt:
3-Sulfamoyl-4-methoxyphenyl-3-propionsäure
   Schmp. 172 - 176°C
3-Sulfamoyl-4-methoxyphenylessigsäure
   Schmp. 164°C

### Herstellung von 3-Sulfonylamino-N-(methylaminothiocarbonyl)-4-methoxyphenylessigsäure

5 g 3-Sulfamoyl-4-methoxy-phenylessigsäure werden in 3 ml DMF gelöst und mit 245 mg Natriumhydroxid für 30 Minuten bei 40°C gerührt. Hierzu gibt man 328 mg Methylisothiocyanat und rührt weitere 2 h bei 70°C. Zu der abgekühlten Lösung gibt man 2N Salzsäure und saugt das Produkt ab. Schmp. 174°C.

### Herstellung von N-5-(1-Phenylethyl)aminocarbonylmethyl-2-methoxysulfamoylbenzol

2,45 g (0.01 Mol) 3-Sulfamoyl-4-methoxyphenylessigsäure und 4,0 g Triethylamin werden in 25 ml DMF gelöst und mit Propanphosphonsäureanhydrid (O.015 Mol; 50%ig in DMF) und nachfolgend mit 1,2 g (0,01 Mol) 1-Phenylethylamin unter Eiskühlung versetzt. Man rührt 3 Stunden bei RT und schüttet die Mischung auf Wasser. Nach einiger Zeit kristallisiert das Produkt und kann vielfach ohne weitere Reinigung für weiteren Umsetzungen verwendet werden.

Analog erhält man
N-5-(1-Phenylethyl)aminocarbonylmethyl-2-ethoxysulfamoylbenzol
N-5-(1-Phenylethyl)aminocarbonylmethyl-2-methylsulfamoylbenzol
N-5-(1-Phenylbutyl)aminocarbonylmethyl-2-ethoxysulfamoylbenzol Schmp.: 156 - 158°C
N-5-(1-Phenylbutyll)aminocarbonylmethyl-2-methylsulfamoylbenzol Schmp.: 117 - 118°C
N-5-(1-Phenylpentyl)aminocarbonylmethyl-2-methoxysulfamoylbenzol Schmp.: 143 - 145°C

Allgemeine Arbeitsvorschrift zur Herstellung der Sulfonyl(thio)harnstoffe 1 aus Sulfonamiden II:
A)
   0.01 Mol eines Sulfonamides II werden in 25 ml DMF gelöst und mit 0.006 Mol K₂CO₃ versetzt. Unter Rühren gibt man 0.011 Mol eines Iso(thio)cyanates hinzu und erwärmt die Mischung etwa 2 - 6 Stunden bei 60 - 80°C. Man gießt die Mischung auf Eiswasser und säuert mit 2 N HCI an. Die ausgefallenen Kristalle werden abgesaugt und gegebenenfalls durch Umkristallisation oder durch Chromatographie an Kieselgel gereinigt. In vielen Fällen ist die Umsetzung jedoch quantitativ bzw das Produkt kristallisiert nach dem Ansäuern in reiner Form aus.
B)
   Zum Sulfonamid II gibt man CS₂ und KOH in DMF, dann zum Dikaliumsalz das Triphosgen. Zum Schluß wird Ammoniak zugegeben und die Lösung wird angesäuert.

### Beispiele:

### Beispiel 1:

N-5-(1-Phenylethyl)aminocarbonylmethyl-2-methoxyphenylsulfonyl-N'-methylthioharnstoff
Schmp.: 175 - 176°C

### Beispiel 2

N-5-(1-Phenylethyl)aminocarbonylmethyl-2-ethoxyphenylsulfonyl-N'-methylthioharnstoff
Schmp.: 156 - 158°C

### Beispiel 3

N-5-(1-Phenylethyl)aminocarbonylmethyl-2-methylphenylsulfonyl-N'-methylthioharnstoff

### Beispiel 4

N-5-(1-Naphthylethyl)aminocarbonylmethyl-2-methoxyphenylsulfonyl-N'-methylthioharnstoff
Schmp.: 188 - 190°C

### Beispiel 5

N-5-(1-Phenylpropyl)aminocarbonylmethyl-2-methoxyphenylsulfonyl-N'-methylthioharnstoff
Schmp.: 125 - 127°C

### Beispiel 6

N-5-(1-Phenylbutyl)aminocarbonylmethyl-2-methoxyphenylsulfonyl-N'-methylthioharnstoff
Schmp.: 128 - 130°C

### Beispiel 7

N-5-(1-Phenylcyclobutylmethyl)aminocarbonylmethyl-2-methoxyphenylsulfonyl-N'-methylthioharnstoff
Schmp. 195 - 197°C

### Beispiel 8

N-5-(1-Phenylcyclohexylmethyl)aminocarbonylmethyl-2-methoxyphenylsulfonyl-N'-methylthioharnstoff
181 - 183°C

### Beispiel 9

N-5-(1-(2-Methoxyphenyl)ethyl)aminocarbonylmethyl-2-methoxyphenylsulfonyl-N'-methylthioharnstoff
Schm.: 178- 179°C

### Beispiel 10

N-5-(1-Phenylpentyl)aminocarbonylmethyl-2-methoxyphenylsulfonyl-N'-methylthioharnstoff
Schm.: 143 - 145°C

### Beispiel 11

N-5-(1-Phenylpropyl)aminocarbonylmethyl-2-methylsulfonyl-N'-methylthioharnstoff
Schmp.: 117 - 118°C

### Beispiel 12

N-5-(1-Phenylbutyl)aminocarbonylmethyl-2-methylsulfonyl-N'-methylthioharnstoff
Schmp.: 112 - 113°C

### Beispiel 13

N-5-(1-Phenylcyclobutylmethyl)aminocarbonylmethyl-2-methylphenylsulfonyl-N'-methylthioharnstoff
Schmp. 130 - 131°C

### Beispiel 14

N-5-(1-Phenylcyclohexylmethyl)aminocarbonylmethyl-2-methylphenylsulfonyl-N'-methylthioharnstoff
Schmp.: 145 - 147°C

### Beispiel 15

N-5-(2-(1,1R-Phenylethyl)aminocarbonyl)ethyl-2-methoxyphenylsulfonyl-N'-methylthioharnstoff
Schmp.: 88°C

### Beispiel 16

N-5-(2-(1,1S-Phenylethyl)aminocarbonyl)ethyl-2-methoxyphenylsulfonyl-N'-methylthioharnstoff
Schmp.: 85°C

### Beispiel 17:

N-5-(1,1 R-Phenylethyl)aminocarbonylmethyl-2-methoxyphenylsulfonyl-N'-methylthioharnstoff
Schmp.: 150 - 152 °C.

### Beispiel 18:

N-5-(1,1S-Phenylethyl)aminocarbonylmethyl-2-methoxyphenylsulfonyl-N'-methylthioharnstoff
Schmp.: 150 - 152 °C.

### Beispiel 19

N-5-(2-(1,1S-Phenylbutyl)aminocarbonyl)ethyl-2-methoxyphenylsulfonyl-N'-methylthioharnstoff
Schmp.: 171 °C

### Pharmakologische Daten:

Mit den folgenden Modellen können die therapeutischen Eigenschaften der Verbindungen I nahegelegt werden:

### (1) Aktionspotentialdauer am Papillarmuskel des Meerschweinchens:

(a) Einleitung
ATP-Mangelzustände, wie sie während einer Ischämie in der Herzmuskelzelle beobachtet werden, führen zu einer Verkürzung der Aktionspotentialdauer. Sie gelten als einer der Ursachen für sogenannte Reentry-Arrhythmien, die den plötzlichen Herztod verursachen können. Die Öffnung von ATP-sensitiven K-Kanälen durch das Absinken von ATP gilt hierfür als ursächlich.
(b) Methode
Zur Messung des Aktionspotentials wird eine Standard-Mikroelektroden-Technik eingesetzt. Hierfür werden Meerschweinchen beiderlei Geschlechts durch Schlag auf den Kopf getötet, die Herzen entnommen, die Papillarmuskeln herausgetrennt und in einem Organbad aufgehängt. Das Organbad wird mit Ringerlösung (0.9% NaCl, 0.048% KCl, 0.024% CaCl₂, 0.02% NaHCO₃, und 0.1% Glucose) durchspült und mit einer Mischung aus 95% Sauerstoff und 5% Kohlendioxid bei einer Temperatur von 36°C begast. Der Muskel wird über eine Elektrode mit Rechteck-Impulsen von 1 V und 1 ms Dauer und einer Frequenz von 2 Hz angeregt. Das Aktionspotential wird durch eine intrazellulär eingestochene Glas-Mikroelektrode, die mit 3 mMol KCl-Lösung gefüllt ist, abgeleitet und registriert. Die zu prüfenden Substanzen wurden der Ringerlösung in einer Konzentration von 2.2·10⁻⁵ Mol pro Liter zugesetzt. Das Aktionspotential wird mit einem Amplifier von Hugo Sachs verstärkt und am Oszilloskop dargestellt. Die Dauer des Aktionspotentials wird bei einem Repolarisierungsgrad von 95% (APD95) bestimmt.
Aktionspotentialverkürzungen werden entweder durch Zugabe einer 1 µM starken Lösung des Kaliumkanalöffners Hoe 234 (J. Kaiser, H. Gögelein, Naunyn-Schmiedebergs Arch. Pharm. 1991, 343, R59) oder durch Zugabe von 2-Desoxyglucose hervorgerufen. Der aktionspotentialverkürzende Effekt dieser Substanzen wurde durch die gleichzeitige Gabe der Testsubstanzen verhindert oder verringert. Testsubstanzen wurden als Stammlösungen in Propandiol der Badlösung zugesetzt. Die angegebenen Werte beziehen sich auf Messungen 30 min nach der Zugabe. Glibenclamid diente in diesen Messungen als Standard. Die Testkonzentration beträgt in allen Fällen 2 x 10⁻⁶ M
(c) Resultate:

| Beispiel Nr. | APD95-Anfang [ms] | APD95 - 30 min [ms] |
|---|---|---|
| 18 | 179 ± 6 | 140 ± 20 |
| 2 | 173 ± 27 | 152 ± 6 |

## Patentansprüche

1. Substituierte Benzolsulfonylharnstoffe und -thioharnstoffe der Formel l, 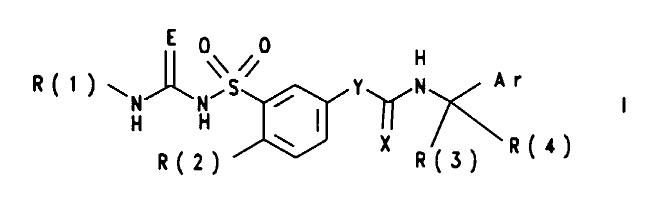 worin bedeuten:
R(1) Wasserstoff, Alkyl mit 1, 2, 3, 4, 5 oder 6 C-Atomen oder Cycloalkyl mit 3, 4, 5 oder 6 C-Atomen;
R(2) (C₁-C₈)-Ketten, in denen ein bis drei C-Atome durch Heteroatome O, NH, S ersetzt sein können, verzweigtes Alkyl mit 1, 2, 3, 4, 5 oder 6 C-Atomen, verzweigtes Alkoxy mit 1, 2, 3, 4, 5 oder 6 C-Atomen oder Cycloalkyl mit 3, 4, 5 oder 6 C-Atomen;
R(3) und R(4) (gleich oder verschieden) Wasserstoff, Alkyl mit 1, 2, 3, 4, 5 oder 6 C-Atomen, oder gemeinsam eine Kette (CH₂)₂₋₅ bilden;
E Sauerstoff oder Schwefel;
X Sauerstoff oder Schwefel;
Y eine Kohlenwasserstoffkette der Formel [CR(5)₂]ₘ;
R(5) Wasserstoff oder Alkyl mit 1 oder 2 C-Atomen;
m 1 oder 2;
Ar Phenyl, Thienyl, Furyl, Pyrrolyl, Thiazolyl, Naphthyl, Pyridyl, das jeweils unsubstituiert oder substituiert ist mit ein bis 3 Substituenten ausgewählt aus der Gruppe bestehend aus Alkyl mit 1 oder 2 C-Atomen, Alkoxy mit 1 oder 2 C-Atomen, Cl, Br oder F;
und ihre physiologisch unbedenklichen Salze.

2. Verbindung der Formel l nach Anspruch 1, worin bedeuten:
R(1) Wasserstoff, Alkyl mit 1, 2, 3, 4, 5 oder 6 C-Atomen oder Cycloalkyl mit 3, 4, 5 oder 6 C-Atomen;
R(2) geradkettiges oder verzweigtes Alkyl mit 1, 2, 3, 4, 5 oder 6 C-Atomen, geradkettiges oder verzweigtes Alkoxy mit 1, 2, 3, 4, 5 oder 6 C-Atomen oder Cycloalkyl mit 3, 4, 5 oder 6 C-Atomen;
R(3) und R(4) (gleich oder verschieden) Wasserstoff, Alkyl mit 1, 2, 3, 4, 5 oder 6 C-Atomen, oder gemeinsam eine Kette (CH₂)₂₋₅ bilden;
E Sauerstoff oder Schwefel;
X Sauerstoff oder Schwefel;
Y eine Kohlenwasserstoffkette der Formel [CR(5)₂]ₘ;
R(5) Wasserstoff oder Alkyl mit 1 oder 2 C-Atomen;
m 1 oder 2;
Ar Phenyl, Thienyl, Furyl, Pyrrolyl, Thiazolyl, Naphthyl, Pyridyl, das jeweils unsubstituiert oder substituiert ist mit ein bis 3 Substituenten ausgewählt aus der Gruppe bestehend aus Alkyl mit 1 oder 2 C-Atomen, Alkoxy mit 1 oder 2 C-Atomen, Cl, Br oder F;
und ihre physiologisch unbedenklichen Salze.

3. Verbindung der Formel l nach Anspruch 1, dadurch gekennzeichnet, daß darin bedeuten:
R(1) Wasserstoff, Alkyl mit 1, 2, 3, 4, 5 oder 6 C-Atomen oder Cycloalkyl mit 3, 4, 5 oder 6 C-Atomen;
R(2) (C₁-C₈)-Ketten, in denen ein bis drei C-Atome durch Heteroatome 0, NH, S ersetzt sein können, verzweigtes Alkyl mit 1, 2, 3, 4, 5 oder 6 C-Atomen, verzweigtes Alkoxy mit 1, 2, 3, 4, 5 oder 6 C-Atomen oder Cycloalkyl mit 3, 4, 5 oder 6 C-Atomen;
R(3) und R(4) (gleich oder verschieden) Wasserstoff, Alkyl mit 1, 2, 3, 4, 5 oder 6 C-Atomen oder gemeinsam eine Kette (CH₂)_{2 - 5} bilden;
E Schwefel;
X Sauerstoff;
Y eine Kohlenwasserstoffkette der Formel [CR(5)₂]₁₋₂,
R(5) Wasserstoff oder Alkyl mit 1 oder 2 C-Atomen;
Ar Phenyl, Thenyl, Furyl, Pyrrolyl, Thiazolyl, Naphthyl, Pyridyl, das jeweils unsubstituiert oder substituiert ist mit ein bis 3 Substituenten ausgewählt aus der Gruppe bestehend aus Alkyl mit 1 oder 2 C-Atomen, Alkoxy mit 1 oder 2 C-Atomen, Cl, Br oder F;
und ihre physiologisch unbedenklichen Salze.

4. Verbindung der Formel l nach einem oder mehreren der Ansprüche 1 bis 3, dadurch gekennzeichnet, daß darin bedeuten:
R(1) Wasserstoff, Alkyl mit 1, 2, 3, 4, 5 oder 6 C-Atomen oder Cycloalkyl mit 3, 4, 5 oder 6 C-Atomen;
R(2) geradkettiges oder verzweigtes Alkyl mit 1, 2, 3, 4, 5 oder 6 C-Atomen, geradkettiges oder verzweigtes Alkoxy mit 1, 2, 3, 4, 5 oder 6 C-Atomen oder Cycloalkyl mit 3, 4, 5 oder 6 C-Atomen;
R(3) und R(4) (gleich oder verschieden) Wasserstoff, Alkyl mit 1, 2, 3, 4, 5 oder 6 C-Atomen oder gemeinsam eine Kette (CH₂)_{2 - 5} bilden;
E Schwefel;
X Sauerstoff;
Y eine Kohlenwasserstoffkette der Formel [CR(5)₂]₁₋₂,
R(5) Wasserstoff oder Alkyl mit 1 oder 2 C-Atomen;
Ar Phenyl, Thenyl, Furyl, Pyrrolyl, Thiazolyl, Naphthyl, Pyridyl, das jeweils unsubstituiert oder substituiert ist mit ein bis 3 Substituenten ausgewählt aus der Gruppe bestehend aus Alkyl mit 1 oder 2 C-Atomen, Alkoxy mit 1 oder 2 C-Atomen, Cl, Br oder F;
und ihre physiologisch unbedenklichen Salze.

5. Verbindung der Formel l nach einem oder mehreren der Ansprüche 1 bis 4, dadurch gekennzeichnet, daß darin bedeuten:
R(1) Wasserstoff oder Alkyl mit 1 oder 2 C-Atomen;
R(2) geradkettiges oder verzweigtes Alkoxy mit 1, 2, 3, 4, 5 oder 6 C-Atomen;
R(3) und R(4) (gleich oder verschieden) Wasserstoff, Alkyl mit 1, 2, 3, 4, 5 oder 6 C-Atomen;
E Schwefel;
X Sauerstoff;
Y eine Kohlenwasserstoffkette der Formel (CH₂)₁₋₂;
Ar Phenyl, Thenyl, Furyl, Pyrrolyl, Thiazolyl, Naphthyl, Pyridyl, das jeweils unsubstituiert oder substituiert ist mit ein bis 3 Substituenten ausgewählt aus der Gruppe bestehend aus Alkyl mit 1 oder 2 C-Atomen, Alkoxy mit 1 oder 2 C-Atomen, Cl, Br und F;
und ihre physiologisch unbedenklichen Salze.

6. Verbindung der Formel l nach einem oder mehreren der Ansprüche 1 bis 5, dadurch gekennzeichnet, daß darin bedeuten:
R(1) Wasserstoff oder Alkyl mit 1 oder 2 C-Atomen;
R(2) geradkettiges oder verzweigtes Alkoxy mit 1, 2, 3, 4, 5 oder 6 C-Atomen;
R(3) und R(4) (gleich oder verschieden) Wasserstoff oder Methyl;
E Schwefel;
X Sauerstoff;
Y eine Kohlenwasserstoffkette der Formel (CH₂)₁₋₂;
Ar Thienyl, Furyl, Pyrrolyl, Thiazolyl, Naphthyl, Pyridyl, das jeweils unsubstituiert oder substituiert ist mit ein bis 3 Substituenten ausgewählt aus der Gruppe bestehend aus Alkyl mit 1 oder 2 C-Atomen, Alkoxy mit 1 oder 2 C-Atomen, Cl, Br oder F;
und ihre physiologisch unbedenklichen Salze.

7. Verbindung der Formel l nach einem oder mehreren der Ansprüche 1 bis 5, dadurch gekennzeichnet, daß darin bedeuten:
R(1) Wasserstoff oder Alkyl mit 1 oder 2 C-Atomen;
R(2) geradkettiges oder verzweigtes Alkoxy mit 1, 2, 3, 4, 5 oder 6 C-Atomen;
R(3) und R(4) (gleich oder verschieden) Wasserstoff oder Methyl;
E Schwefel;
X Sauerstoff;
Y eine Kohlenwasserstoffkette der Formel (CH₂)₁₋₂;
Ar Phenyl, das unsubstituiert oder substituiert ist mit ein bis 3 Substituenten ausgewählt aus der Gruppe bestehend aus Alkyl mit 1 oder 2 C-Atomen, Alkoxy mit 1 oder 2 C-Atomen, Cl, Br oder F;
und ihre physiologisch unbedenklichen Salze.

8. Verfahren zur Herstellung einer Verbindung der Formel l nach einem oder mehreren der Ansprüche 1 bis 7, dadurch gekennzeichnet, daß man
(a) ein aromatisches Sulfonamid der Formel II 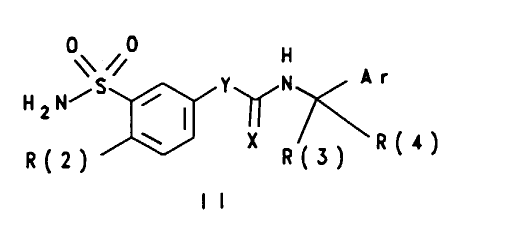 oder dessen Salz der Formel III 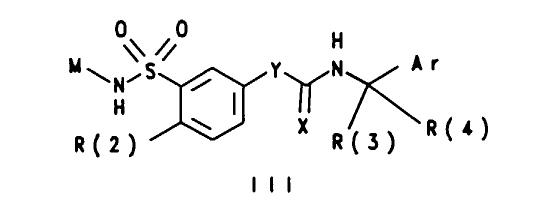 worin R(2), R(3), R(4) und Ar die in den Ansprüchen 1 bis 7 angegebenen Bedeutungen haben und das Kation M für ein Alkali-, Erdalkali-, Ammonium- oder Tetraalkylammoniumion steht,
mit einem R(1)-substituierten Isocyanat der Formel IV,
R(1)-N=C=O IV
worin R(1) die in den Ansprüchen 1 bis 7 angegebene Bedeutung hat, zu substituierten Benzolsulfonylharnstoffen der Formel l a (E = Sauerstoff) 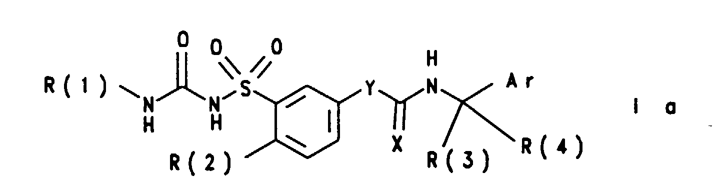 umsetzt;
oder
(b) daß man unsubstituierte Benzolsulfonylharnstoffe der Formel l a [R(1) = H, E = O) durch Umsetzung eines aromatischen Benzolsulfonamids der Formel II oder von dessen Salz der Formel III mit Trialkylsilylisocyanat oder Siliciumtetraisocyanat und Spaltung (z. B. Hydrolyse) der primären siliciumsubstituierten Benzolsulfonylharnstoffe herstellt;
oder daß man
(c) einen Benzolsulfonylharnstoff der Formel l a (E = Sauerstoff) aus einem aromatischen Benzolsulfonamid der Formel II oder dessen Salz der Formel III mit einem R(1)-substituierten Trichloracetamid der Formel V 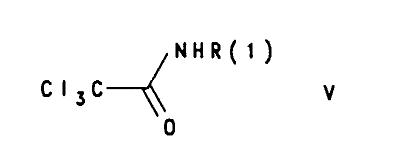 in Gegenwart einer Base in einem inerten Lösungsmittel darstellt; oder daß man
(d) einen Benzolsulfonylthioharnstoff der Formel l b (E = S) 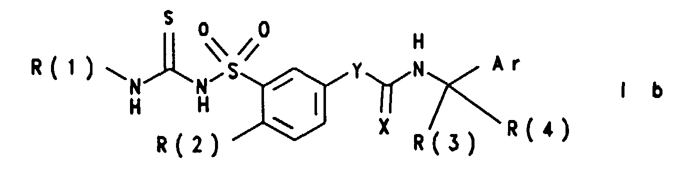 aus einem Benzolsulfonamid der Formel II oder dessen Salz der Formel III und einem R(1)-substituierten Isothiocyanat der Formel VI
R(1)-N=C=S VI
herstellt;
oder daß man
(e) einen substituierten Benzolsulfonylharnstoff der Formel l a (E = Sauerstoff) durch eine Umwandlungsreaktion aus einem Benzolsulfonylthioharnstoff der Formel l b (E = S) herstellt;
oder daß man
(f) einen Benzolsulfonylharnstoff der Formel l aus einem Benzolsulfonylhalogenid der Formel VII 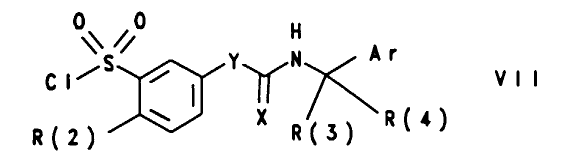 mit einem R(1)-substituierten Harnstoff oder einem R(1)-substituierten Bis(trialkylsilyl)harnstoff herstellt;
oder daß man
(g) einen Benzolsulfonylharnstoff der Formel I a durch Umsetzung eines Amins der Formel R(1)-NH₂ mit einem Benzolsulfonylisocyanat der Formel VIII 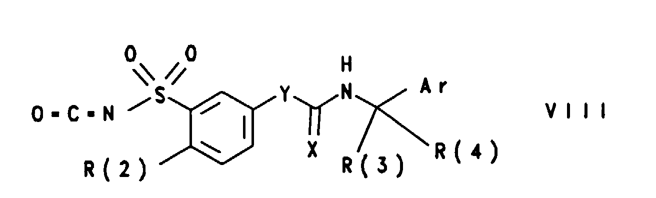 herstellt;
oder daß man
(h) einen Benzolsulfonylthioharnstoff der Formel I b durch Umsetzen von einem Amin der Formel R(1)-NH₂ mit einem Benzolsulfonylisothiocyanat der Formel IX 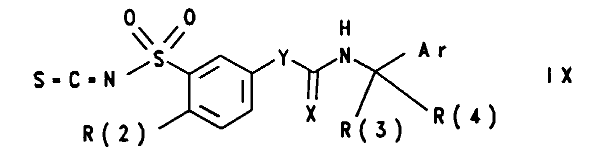 herstellt;
oder daß man
(i) einen Benzolsulfenyl- oder -sulfinylharnstoff mit einem Oxidationsmittel zum Benzolsulfonylharnstoff der Formel I a oxidiert.

9. Verwendung einer Verbindung der Formel I nach einem oder mehreren der Ansprüche 1 bis 7 oder eines physiologisch unbedenklichen Salzes davon zur Herstellung eines Medikaments zur Behandlung von Herzrhythmusstörungen.

10. Verwendung einer Verbindung der Formel I nach einem oder mehreren der Ansprüche 1 bis 7 oder eines physiologisch unbedenklichen Salzes davon zur Herstellung eines Medikaments zur Verhinderung des plötzlichen Herztods.

11. Verwendung einer Verbindung der Formel l nach einem oder mehreren der Ansprüche 1 bis 7 oder eines physiologisch unbedenklichen Salzes davon zur Herstellung eines Medikaments zur Behandlung von ischämischen Zuständen des Herzens.

12. Verwendung einer Verbindung der Formel l nach einem oder mehreren der Ansprüche 1 bis 7 oder eines physiologisch unbedenklichen Salzes davon zur Herstellung eines Medikaments zur Behandlung der geschwächten Herzkraft.

13. Verwendung einer Verbindung der Formel l nach einem oder mehreren der Ansprüche 1 bis 7 oder eines physiologisch unbedenklichen Salzes davon zur Herstellung eines Medikaments zur Verbesserung der Herzfunktion nach Herztransplantation.

14. Heilmittel, gekennzeichnet durch eine wirksame Menge an einer Verbindung der Formel l nach einem oder mehreren der Ansprüche 1 bis 7 oder eines physiologisch unbedenklichen Salzes davon.

## Claims

1. A substituted benzenesulfonylurea or -thiourea of the formula I 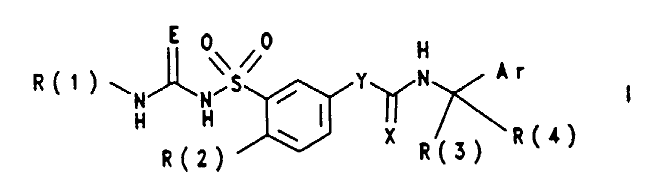 in which:
R(1) is hydrogen, alkyl having 1, 2, 3, 4, 5 or 6 carbon atoms or cycloalkyl having 3, 4, 5 or 6 carbon atoms;
R(2) is (C₁-C₈) chains in which one to three carbon atoms can be replaced by heteroatoms selected from the group consisting of O, NH and S, branched alkyl having 1, 2, 3, 4, 5 or 6 carbon atoms, branched alkoxy having 1, 2, 3, 4, 5 or 6 carbon atoms or cycloalkyl having 3, 4, 5 or 6 carbon atoms;
R(3) and R(4) (identical or different) are hydrogen, alkyl having 1, 2, 3, 4, 5 or 6 carbon atoms, or together form a (CH₂)₂₋₅ chain;
E is oxygen or sulfur;
X is oxygen or sulfur;
Y is a hydrocarbon chain of the formula (CR(5)₂]ₘ;
R(5) is hydrogen or alkyl having 1 or 2 carbon atoms;
m is 1 or 2;
Ar is phenyl, thienyl, furyl, pyrrolyl, thiazolyl, naphthyl, pyridyl, which in each case is unsubstituted or substituted by one to 3 substituents selected from the group consisting of alkyl having 1 or 2 carbon atoms, alkoxy having 1 or 2 carbon atoms, Cl, Br and F; and its physiologically acceptable salts.

2. A compound of the formula I as claimed in claim 1, wherein:
R(1) is hydrogen, alkyl having 1, 2, 3, 4, 5 or 6 carbon atoms or cycloalkyl having 3, 4, 5 or 6 carbon atoms;
R(2) is straight-chain or branched alkyl having 1, 2, 3, 4, 5 or 6 carbon atoms, straight-chain or branched alkoxy having 1, 2, 3, 4, 5 or 6 carbon atoms or cycloalkyl having 3, 4, 5 or 6 carbon atoms;
R(3) and R(4) (identical or different) are hydrogen, alkyl having 1, 2, 3, 4, 5 or 6 carbon atoms or together form a (CH₂)₂₋₅ chain;
E is oxygen or sulfur;
X is oxygen or sulfur;
Y is a hydrocarbon chain of the formula [CR(5)₂]ₘ,
R(5) is hydrogen or alkyl having 1 or 2 carbon atoms;
m is 1 or 2;
Ar is phenyl, thienyl, furyl, pyrrolyl, thiazolyl, naphthyl, pyridyl, which in each case is unsubstituted or substituted by one to 3 substituents selected from the group consisting of alkyl having 1 or 2 carbon atoms, alkoxy having 1 or 2 carbon atoms, Cl, Br and F; and its physiologically acceptable salts.

3. A compound of the formula I as claimed in claim 1, wherein:
R(1) is hydrogen or alkyl having 1, 2, 3, 4, 5 or 6 carbon atoms or cycloalkyl having 3, 4, 5 or 6 carbon atoms;
R(2) is (C₁-C₈) chains in which one to three carbon atoms can be replaced by heteroatoms selected from the group consisting of O, NH and S, branched alkyl having 1, 2, 3, 4, 5 or 6 carbon atoms, branched alkoxy having 1, 2, 3, 4, 5 or 6 carbon atoms or cycloalkyl having 3, 4, 5 or 6 carbon atoms;
R(3) and R(4) (identical or different) are hydrogen, alkyl having 1, 2, 3, 4, 5 or 6 carbon atoms or together form a (CH₂)₂₋₅ chain;
E is sulfur;
X is oxygen;
Y is a hydrocarbon chain of the formula (CR(5)₂]₁₋₂;
R(5) is hydrogen or alkyl having 1 or 2 carbon atoms;
Ar is phenyl, thenyl, furyl, pyrrolyl, thiazolyl, naphthyl, pyridyl, which in each case is unsubstituted or substituted by one to 3 substituents selected from the group consisting of alkyl having 1 or 2 carbon atoms, alkoxy having 1 or 2 carbon atoms, Cl, Br and F; and its physiologically acceptable salts.

4. A compound of the formula I as claimed in one or more of claims 1 to 3, wherein:
R(1) is hydrogen, alkyl having 1, 2, 3, 4, 5 or 6 carbon atoms or cycloalkyl having 3, 4, 5 or 6 carbon atoms;
R(2) is straight-chain or branched alkyl having 1, 2, 3, 4, 5 or 6 carbon atoms, straight-chain or branched alkoxy having 1, 2, 3, 4, 5 or 6 carbon atoms or cycloalkyl having 3, 4, 5 or 6 carbon atoms;
R(3) and R(4) (identical or different) are hydrogen, alkyl having 1, 2, 3, 4, 5 or 6 carbon atoms or together form a (CH₂)₂₋₅ chain;
E is sulfur;
X is oxygen;
Y is a hydrocarbon chain of the formula (CR(5)₂]₁₋₂;
R(5) is hydrogen or alkyl having l or 2 carbon atoms;
Ar is phenyl, thenyl, furyl, pyrrolyl, thiazolyl, naphthyl, pyridyl, which in each case is unsubstituted or substituted by one to 3 substituents selected from the group consisting of alkyl having 1 or 2 carbon atoms, alkoxy having 1 or 2 carbon atoms, Cl, Br and F; and its physiologically acceptable salts.

5. A compound of the formula I as claimed in one or more of claims 1 to 4, wherein:
R(1) is hydrogen or alkyl having 1 or 2 carbon atoms;
R(2) is straight-chain or branched alkoxy having 1, 2, 3, 4, 5 or 6 carbon atoms;
R(3) and R(4) (identical or different) are hydrogen or alkyl having 1, 2, 3, 4, 5 or 6 carbon atoms;
E is sulfur;
X is oxygen;
Y is a hydrocarbon chain of the formula (CH₂)₁₋₂;
Ar is phenyl, thenyl, furyl, pyrrolyl, thiazolyl, naphthyl, pyridyl which in each case is unsubstituted or substituted by one to 3 substituents selected from the group consisting of alkyl having 1 or 2 carbon atoms, alkoxy having 1 or 2 carbon atoms, Cl, Br and F; and
its physiologically acceptable salts.

6. A compound of the formula I as claimed in one or more of claims 1 to 5, wherein:
R(1) is hydrogen or alkyl having 1 or 2 carbon atoms;
R(2) is straight-chain or branched alkoxy having 1, 2, 3, 4, 5 or 6 carbon atoms;
R(3) and R(4) (identical or different) are hydrogen or methyl;
E is sulfur;
X is oxygen;
Y is a hydrocarbon chain of the formula (CH₂)₁₋₂;
Ar is thienyl, furyl, pyrrolyl, thiazolyl, naphthyl, pyridyl, which in each case is unsubstituted or substituted by one to 3 substituents selected from the group consisting of alkyl having 1 or 2 carbon atoms, alkoxy having 1 or 2 carbon atoms, Cl, Br and F;
and its physiologically acceptable salts.

7. A compound of the formula I as claimed in one or more of claims 1 to 5, wherein:
R(1) is hydrogen or alkyl having 1 or 2 carbon atoms;
R(2) is straight-chain or branched alkoxy having 1, 2, 3, 4, 5 or 6 carbon atoms;
R(3) and R(4) (identical or different) are hydrogen or methyl;
E is sulfur;
X is oxygen;
Y is a hydrocarbon chain of the formula (CH₂)₁₋₂;
Ar is phenyl which is unsubstituted or substituted by one to 3 substituents selected from the group consisting of alkyl having 1 or 2 carbon atoms, alkoxy having 1 or 2 carbon atoms, Cl, Br and F;
and its physiologically acceptable salts.

8. A process for the preparation of a compound of the formula I as claimed in one or more of claims l to 7, which comprises
(a) reacting an aromatic sulfonamide of the formula II 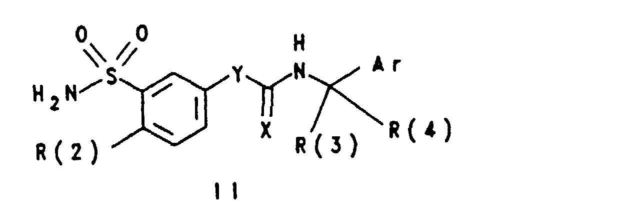 or its salt of the formula III 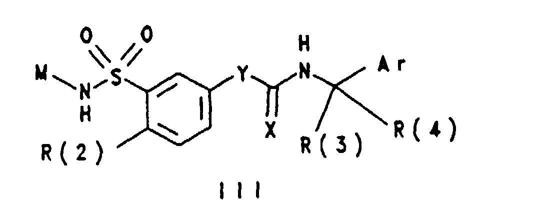 in which R(2), R(3), R(4) and Ar have the meanings indicated in claims l to 7, and the cation M is an alkali metal, alkaline earth metal, ammonium or tetraalkylammonium ion,
with an R(1)-substituted isocyanate of the formula IV
R(1)-N=C=O IV
in which R(1) has the meaning indicated in claims 1 to 7, to give substituted benzenesulfonylureas of the formula I a (E = oxygen) 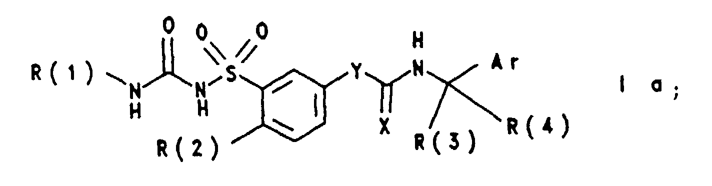 or
(b) preparing unsubstituted benzenesulfonylureas of the formula I a [R(1) = H, E = O] by reaction of an aromatic benzenesulfonamide of the formula II or of its salt of the formula III with trialkylsilyl isocyanate or silicon tetraisocyanate and cleavage (e.g. hydrolysis) of the primary silicon-substituted benzenesulfonylureas;
or
(c) preparing a benzenesulfonylurea of the formula I a (E = oxygen) from an aromatic benzenesulfonamide of the formula II or its salt of the formula III using an R(1)-substituted trichloroacetamide of the formula V 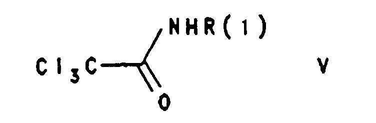 in the presence of a base in an inert solvent;
or
(d) preparing a benzenesulfonylthiourea of the formula I b (E = S) 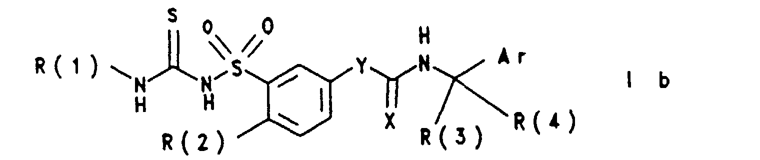 from a benzenesulfonamide of the formula II or its salt of the formula III and an R(1)-substituted isothiocyanate of the formula VI
R(1)-N=C=S VI;
or
(e) preparing a substituted benzenesulfonylurea of the formula I a (E = oxygen) by a conversion reaction of a benzenesulfonylthiourea of the formula I b (E=S) ;
or
(f) preparing a benzenesulfonylurea of the formula I from a benzenesulfonyl halide of the formula VII 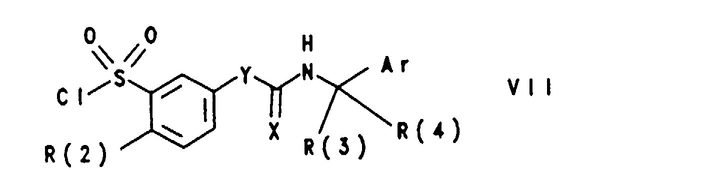 using an R(1)-substituted urea or an R(1)-substituted bis(trialkylsilyl)urea;
or
(g) preparing a benzenesulfonylurea of the formula I a by reaction of an amine of the formula R(1)-NH2 with a benzenesulfonyl isocyanate of the formula VIII 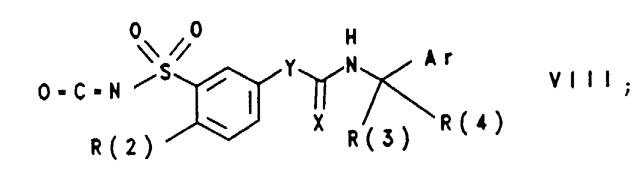 or
(h) preparing a benzenesulfonylthiourea of the formula I b by reaction of an amine of the formula R(1)-NH₂ with a benzenesulfonyl isothiocyanate of the formula IX 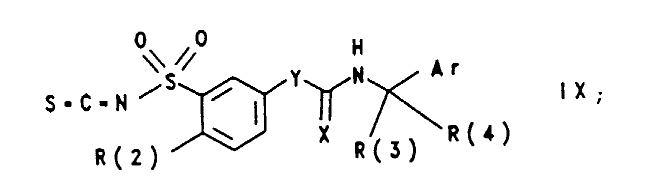 or
(i) oxidizing a benzenesulfenyl- or -sulfinylurea using an oxidant to give the benzenesulfonylurea of the formula I a.

9. The use of a compound of the formula I as claimed in one or more of claims 1 to 7 or of a physiologically acceptable salt thereof for the production of a medicament for the treatment of cardiac arrhythmias.

10. The use of a compound of the formula I as claimed in one or more of claims 1 to 7 or of a physiologically acceptable salt thereof for the production of a medicament for the prevention of sudden heart death.

11. The use of a compound of the formula I as claimed in one or more of claims 1 to 7 or of a physiologically acceptable salt thereof for the production of a medicament for the treatment of ischemic conditions of the heart.

12. The use of a compound of the formula I as claimed in one of more of claims 1 to 7 or of a physiologically acceptable salt thereof for the production of a medicament for the treatment of weakened cardiac power.

13. The use of a compound of the formula I as claimed in one or more of claims 1 to 7 or of a physiologically acceptable salt thereof for the production of a medicament for the improvement of heart function after heart transplantation.

14. A medicament comprising an effective amount of a compound of the formula I as claimed in one or more of claims 1 to 7 or of a physiologically acceptable salt thereof.

## Revendications

1. Benzènesulfonylurées et -thio-urées substituées de formule I 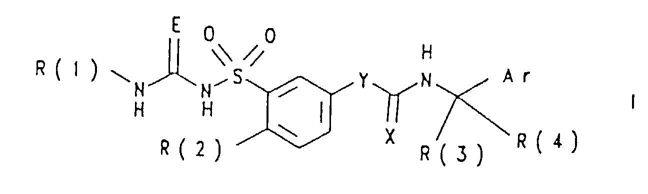 dans laquelle
R(1) représente un atome d'hydrogène ou un groupe alkyle ayant 1, 2, 3, 4, 5 ou 6 atomes de carbone, ou cycloalkyle ayant 3, 4, 5 ou 6 atomes de carbone;
R(2) représente des chaînes en C₁-c₈ dans lesquelles un à trois atomes de carbone peuvent être remplacés par des hétéroatomes O, NH, S, un groupe alkyle ramifié ayant 1, 2, 3, 4, 5 ou 6 atomes de carbone, alcoxy ramifié ayant 1, 2, 3, 4, 5 ou 6 atomes de carbone ou cycloalkyle ayant 3, 4, 5 ou 6 atomes de carbone;
R(3) et R(4) (identiques ou différents) représentent un atome d'hydrogène ou un groupe alkyle ayant 1, 2, 3, 4, 5 ou 6 atomes de carbone, ou forment ensemble une chaîne (CH₂)₂₋₅;
E représente un atome d'oxygène ou de soufre;
X représente un atome d'oxygène ou de soufre;
Y représente une chaîne hydrocarbonée de formule [CR(5)₂]ₘ;
R(5) étant un atome d'hydrogène ou un groupe alkyle ayant 1 ou 2 atomes de carbone;
m étant 1 ou 2;
Ar représente un groupe phényle, thiényle, furyle, pyrrolyle, thiazolyle, naphtyle ou pyridyle qui, dans chaque cas, est non substitué ou porte un à trois substituants choisis dans l'ensemble constitué par des groupes alkyle ayant 1 ou 2 atomes de carbone, des groupes alcoxy ayant 1 ou 2 atomes de carbone, Cl, Br ou F;
et leurs sels physiologiquement acceptables.

2. Composé de formule I selon la revendication 1, dans lequel:
R(1) représente un atome d'hydrogène ou un groupe alkyle ayant 1, 2, 3, 4, 5 ou 6 atomes de carbone, ou cycloalkyle ayant 3, 4, 5 ou 6 atomes de carbone;
R(2) représente un groupe alkyle à chaîne droite ou ramifié ayant 1, 2, 3, 4, 5 ou 6 atomes de carbone, alcoxy à chaîne droite ou ramifié ayant 1, 2, 3, 4, 5 ou 6 atomes de carbone ou cycloalkyle ayant 3, 4, 5 ou 6 atomes de carbone;
R(3) et R(4) (identiques ou différents) représentent un atome d'hydrogène ou un groupe alkyle ayant 1, 2, 3, 4, 5 ou 6 atomes de carbone, ou forment ensemble une chaîne (CH₂)₂₋₅;
E représente un atome d'oxygène ou de soufre;
X représente un atome d'oxygène ou de soufre;
Y représente une chaîne hydrocarbonée de formule [CR(5)₂]ₘ;
R(5) étant un atome d'hydrogène ou un groupe alkyle ayant 1 ou 2 atomes de carbone;
m étant 1 ou 2;
Ar représente un groupe phényle, thiényle, furyle, pyrrolyle, thiazolyle, naphtyle ou pyridyle qui, dans chaque cas, est non substitué ou porte un à trois substituants choisis dans l'ensemble constitué par des groupes alkyle ayant 1 ou 2 atomes de carbone, des groupes alcoxy ayant 1 ou 2 atomes de carbone, Cl, Br ou F;
et ses sels physiologiquement acceptables.

3. Composé de formule I selon la revendication 1, caractérisé en ce que dans cette formule:
R(1) représente un atome d'hydrogène ou un groupe alkyle ayant 1, 2, 3, 4, 5 ou 6 atomes de carbone, ou cycloalkyle ayant 3, 4, 5 ou 6 atomes de carbone;
R(2) représente des chaînes en C₁-C₈ dans lesquelles un à trois atomes de carbone peuvent être remplacés par des hétéroatomes O, NH, S, un groupe alkyle ramifié ayant 1, 2, 3, 4, 5 ou 6 atomes de carbone, alcoxy ramifié ayant 1, 2, 3, 4, 5 ou 6 atomes de carbone ou cycloalkyle ayant 3, 4, 5 ou 6 atomes de carbone;
R(3) et R(4) (identiques ou différents) représentent un atome d'hydrogène ou un groupe alkyle ayant 1, 2, 3, 4, 5 ou 6 atomes de carbone, ou forment ensemble une chaîne (CH₂)₂₋₅;
E représente un atome de soufre;
X représente un atome d'oxygène;
Y représente une chaîne hydrocarbonée de formule [CR(5)₂]₁₋₂;
R(5) étant un atome d'hydrogène ou un groupe alkyle ayant 1 ou 2 atomes de carbone;
Ar représente un groupe phényle, thényle, furyle, pyrrolyle, thiazolyle, naphtyle ou pyridyle qui, dans chaque cas, est non substitué ou porte un à trois substituants choisis dans l'ensemble constitué par des groupes alkyle ayant 1 ou 2 atomes de carbone, des groupes alcoxy ayant 1 ou 2 atomes de carbone, Cl, Br ou F;
et ses sels physiologiquement acceptables.

4. Composé de formule I selon une ou plusieurs des revendications 1 à 3, caractérisé en ce que, dans cette formule:
R(1) représente un atome d'hydrogène ou un groupe alkyle ayant 1, 2, 3, 4, 5 ou 6 atomes de carbone, ou cycloalkyle ayant 3, 4, 5 ou 6 atomes de carbone;
R(2) représente un groupe alkyle à chaîne droite ou ramifié ayant 1, 2, 3, 4, 5 ou 6 atomes de carbone, alcoxy à chaîne droite ou ramifié ayant 1, 2, 3, 4, 5 ou 6 atomes de carbone ou cycloalkyle ayant 3, 4, 5 ou 6 atomes de carbone;
R(3) et R(4) (identiques ou différents) représentent un atome d'hydrogène ou un groupe alkyle ayant 1, 2, 3, 4, 5 ou 6 atomes de carbone, ou forment ensemble une chaîne (CH₂)₂₋₅;
E représente un atome de soufre;
X représente un atome d'oxygène;
Y représente une chaîne hydrocarbonée de formule [CR(5)₂]₁₋₂;
R(5) étant un atome d'hydrogène ou un groupe alkyle ayant 1 ou 2 atomes de carbone;
Ar représente un groupe phényle, thényle, furyle, pyrrolyle, thiazolyle, naphtyle ou pyridyle qui, dans chaque cas, est non substitué ou porte un à trois substituants choisis dans l'ensemble constitué par des groupes alkyle ayant 1 ou 2 atomes de carbone, des groupes alcoxy ayant 1 ou 2 atomes de carbone, Cl, Br ou F;
et ses sels physiologiquement acceptables.

5. Composé de formule I selon une ou plusieurs des revendications 1 à 4, caractérisé en ce que dans cette formule:
R(1) représente un atome d'hydrogène ou un groupe alkyle ayant 1 ou 2 atomes de carbone;
R(2) représente un groupe alcoxy à chaîne droite ou ramifié ayant 1, 2, 3, 4, 5 ou 6 atomes de carbone;
R(3) et R(4) (identiques ou différents) représentent un atome d'hydrogène ou un groupe alkyle ayant 1, 2, 3, 4, 5 ou 6 atomes de carbone;
E représente un atome de soufre;
X représente un atome d'oxygène;
Y représente une chaîne hydrocarbonée de formule (CH₂)₁₋₂;
Ar représente un groupe phényle, thényle, furyle, pyrrolyle, thiazolyle, naphtyle ou pyridyle qui, dans chaque cas, est non substitué ou porte un à trois substituants choisis dans l'ensemble constitué par des groupes alkyle ayant 1 ou 2 atomes de carbone, des groupes alcoxy ayant 1 ou 2 atomes de carbone, Cl, Br ou F;
et ses sels physiologiquement acceptables.

6. Composé de formule I selon une ou plusieurs des revendications 1 à 5, caractérisé en ce que dans cette formule:
R(1) représente un atome d'hydrogène ou un groupe alkyle ayant 1 ou 2 atomes de carbone;
R(2) représente un groupe alcoxy à chaîne droite ou ramifié ayant 1, 2, 3, 4, 5 ou 6 atomes de carbone;
R(3) et R(4) (identiques ou différents) représentent un atome d'hydrogène ou le groupe méthyle;
E représente un atome de soufre;
X représente un atome d'oxygène;
Y représente une chaîne hydrocarbonée de formule (CH₂)₁₋₂;
Ar représente un groupe thiényle, furyle, pyrrolyle, thiazolyle, naphtyle ou pyridyle qui, dans chaque cas, est non substitué ou porte un à trois substituants choisis dans l'ensemble constitué par des groupes alkyle ayant 1 ou 2 atomes de carbone, des groupes alcoxy ayant 1 ou 2 atomes de carbone, Cl, Br ou F;
et ses sels physiologiquement acceptables.

7. Composé de formule I selon une ou plusieurs des revendications 1 à 5, caractérisé en ce que dans cette formule:
R(1) représente un atome d'hydrogène ou un groupe alkyle ayant 1 ou 2 atomes de carbone;
R(2) représente un groupe alcoxy à chaîne droite ou ramifié ayant 1, 2, 3, 4, 5 ou 6 atomes de carbone;
R(3) et R(4) (identiques ou différents) représentent un atome d'hydrogène ou le groupe méthyle;
E représente un atome de soufre;
X représente un atome d'oxygène;
Y représente une chaîne hydrocarbonée de formule (CH₂)₁₋₂;
Ar représente un groupe phényle qui est non substitué ou porte un à trois substituants choisis dans l'ensemble constitué par des groupes alkyle ayant 1 ou 2 atomes de carbone, des groupes alcoxy ayant 1 ou 2 atomes de carbone, Cl, Br ou F;
et ses sels physiologiquement acceptables.

8. Procédé pour la préparation d'un composé de formule I selon une ou plusieurs des revendications 1 à 7,
caractérisé en ce que
(a) on fait réagir un sulfonamide aromatique de formule II 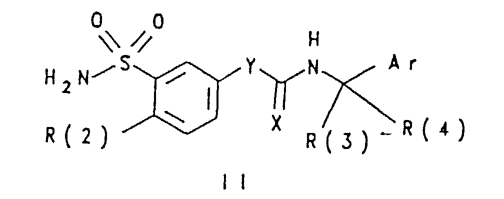 ou un sel de celui-ci, de formule III 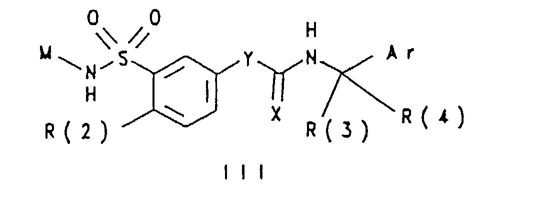 formules dans lesquelles R(2), R(3), R(4) et Ar ont les significations données dans les revendications 1 à 7, et le cation M représente un ion alcalin, alcalino-terreux, ammonium ou tétraalkylammonium,
avec un isocyanate substitué par R(1), de formule IV
R(1)-N=C=O IV
dans laquelle R(1) a la signification donnée dans les revendications 1 à 7,
pour aboutir à des benzènesulfonylurées substituées de formule Ia (E = oxygène), 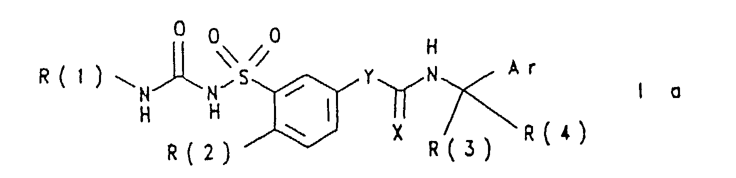 ou en ce que
(b) on prépare des benzènesulfonylurées non substituées de formule Ia [R(1) = H, E = O] par la réaction d'un benzènesulfonamide aromatique de formule II ou d'un sel de celui-ci de formule III, avec un trialkylsilylisocyanate ou du tétra-isocyanate de silicium, et la coupure (par exemple l'hydrolyse) des benzènesulfonylurées primaires substituées par le silicium;
ou en ce que
(c) on prépare une benzènesulfonylurée de formule Ia (E = oxygène) à partir d'un benzènesulfonamide aromatique de formule II ou d'un de ses sels de formule III, avec un trichloracétamide substitué par R(1), de formule V 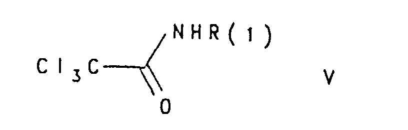 en présence d'une base, dans un solvant inerte;
ou en ce que
(d) on prépare une benzènesulfonylthio-urée Ib (E = S) 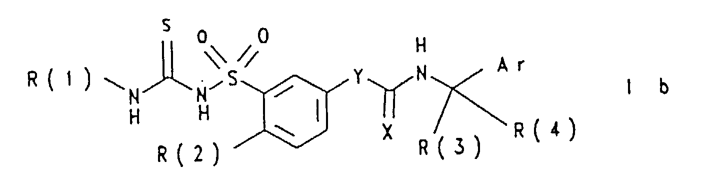 à partir d'un benzènesulfonamide de formule II ou d'un de ses sels de formule III et d'un isothiocyanate substitué par R(1), de formule VI
R(1)-N=C=S VI
ou en ce que
(e) on prépare une benzènesulfonylurée substituée de formule Ia (E = oxygène) par une réaction de conversion à partir d'une benzènesulfonylthio-urée de formule Ib (E = S);
ou en ce que
(f) on prépare une benzènesulfonylurée de formule I à partir d'un halogénure de benzènesulfonyle de formule VII 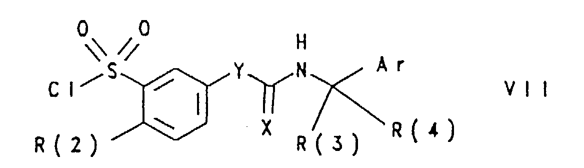 avec une urée substituée par R(1) ou une bis(trialkylsilyl)urée substituée par R(1);
ou en ce que
(g) on prépare une benzènesulfonylurée de formule Ia par la réaction d'une amine de formule R(1)-NH₂ avec un benzènesulfonylisocyanate de formule VIII 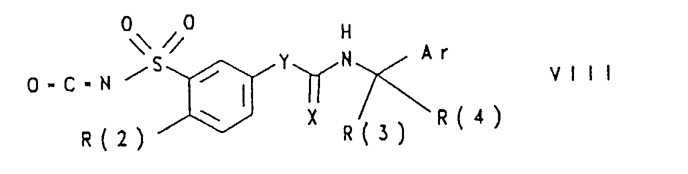 ou en ce que
(h) on prépare une benzènesulfonylthio-urée de formule Ib par la réaction d'une amine de formule R(1)-NH₂ avec un benzènesulfonylisothiocyanate de formule IX 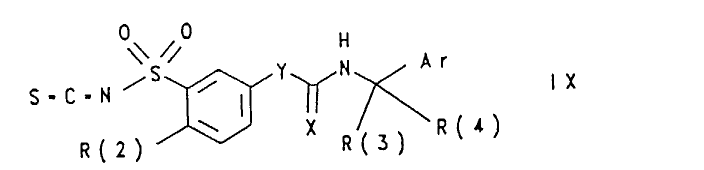 ou en ce que
(i) on oxyde avec un oxydant une benzènesulfényl- ou -sulfinylurée en la benzènesulfonylurée de formule Ia.

9. Utilisation d'un composé de formule I selon une ou plusieurs des revendications 1 à 7, ou d'un sel physiologiquement acceptable de celui-ci, pour la fabrication d'un médicament destiné au traitement de troubles du rythme cardiaque.

10. Utilisation d'un composé de formule I selon une ou plusieurs des revendications 1 à 7, ou d'un sel physiologiquement acceptable de celui-ci, pour la fabrication d'un médicament destiné à empêcher la mort subite par arrêt cardiaque.

11. Utilisation d'un composé de formule I selon une ou plusieurs des revendications 1 à 7, ou d'un sel physiologiquement acceptable de celui-ci, pour la fabrication d'un médicament destiné au traitement d'états ischémiques du coeur.

12. Utilisation d'un composé de formule I selon une ou plusieurs des revendications l à 7, ou d'un sel physiologiquement acceptable de celui-ci, pour la fabrication d'un médicament destiné au traitement de la force cardiaque affaiblie.

13. Utilisation d'un composé de formule I selon une ou plusieurs des revendications 1 à 7, ou d'un sel physiologiquement acceptable de celui-ci, pour la fabrication d'un médicament destiné à l'amélioration de la fonction cardiaque après une greffe du coeur.

14. Médicament, caractérisé par une quantité efficace d'un composé de formule I selon une ou plusieurs des revendications 1 à 7, ou d'un sel physiologiquement acceptable d'un tel composé.
